# EUROPEAN PATENT SPECIFICATION

(11) **EP 4 452 267 B1**
(45) Date of publication and mention of the grant of the patent: **23.04.2025**
(21) Application number: 22844112.7
(22) Date of filing: 23.12.2022
(51) Int. Cl.: A61K 31/4418, A61P 27/16, A61K 9/00

(54) **4-PHENYL-TETRAHYDROPYRIDINE DERIVATIVES FOR TREATING HEARING DISEASES**
4-PHENYL-TETRAHYDROPYRIDIN-DERIVATE ZUR BEHANDLUNG VON GEHÖRERKRANKUNGEN
DÉRIVÉS 4-PHÉNYL-TÉTRAHYDROPYRIDINE POUR LE TRAITEMENT DE MALADIES AUDITIVES

(30) Priority: 23.12.2021 EP 21306931
(43) Date of publication of application: 30.10.2024
(60) Divisional application: 25163348.3
(73) Proprietor: Cilcare Dev, 34080 Montpellier (FR)
(72) Inventor: BELLINE, Célia, 34080 Montpellier (FR); PASDELOU, Marie-Pierre, 34080 Montpellier (FR); PUCHEU, Sylvie, 34080 Montpellier (FR); NAERT, Gaëlle, 34080 Montpellier (FR); SAGE, Cyrille, 34080 Montpellier (FR); SCHUE, Mathieu, 34080 Montpellier (FR)
(74) Representative: Icosa
(86) International application number: PCT/EP2022/087667
(87) International publication number: WO 2023/118544

(56) References cited:
- EP-A2- 1 263 504
- LEMAIRE L ET AL: "MAGNETIC RESONANCE IMAGING OF THE NEUROPROTECTIVE EFFECT OF XALIPRODEN IN RATS", INVESTIGATIVE RADIOLOGY, PHILADELPHIA, PA, US, vol. 37, no. 6, 1 June 2002 (2002-06-01), pages 321 - 327, XP008024309, DOI: 10.1097/00004424-200206000-00003

## Description

### FIELD OF INVENTION

The present invention relates to 4-phenyl-tetrahydropyridine derivatives for use in the treatment of hearing diseases, in particular in the treatment of unilateral or bilateral sensorineural hearing loss and/or of unilateral or bilateral cochlear synaptopathy, optionally combined with unilateral or bilateral tinnitus. The present invention also relates to a pharmaceutical composition allowing a trans-tympanic administration of 4-phenyl-tetrahydropyridine derivatives and to said pharmaceutical composition for use in the treatment of hearing diseases, in particular in the treatment of unilateral or bilateral sensorineural hearing loss and/or of unilateral or bilateral cochlear synaptopathy, optionally combined with unilateral or bilateral tinnitus.

### BACKGROUND OF INVENTION

Among the hearing diseases, sensorineural hearing loss (SNHL) is the most common type of permanent hearing loss, accounting for about 90% of reported hearing loss. In most cases, SNHL results from ageing (presbycusis) or exposition either to excessive noise levels or to ototoxic chemicals, causing damage to inner and outer hair cells and leading to transient or permanent hearing loss (increase of hearing thresholds). There is currently no available pharmacological treatment to SNHL and hearing medical devices (such as hearing aids or cochlear implants) are the only solution helping the patient to hear.

Besides the destruction of cochlear hair cells, over-exposition to noise can also cause disruption of synaptic connections between inner hair cells and auditory nerve fibers of spiral ganglion neurons, known as excitotoxicity. Then, disconnected spiral ganglion neurons gradually die and disappear over time. The consequence is a cochlear synaptopathy, which may be either a hidden form of hearing loss or associated with a hearing loss. Patients suffering from cochlear synaptopathy have a speech-in-noise intelligibility deficit. In addition, cochlear synaptopathy is often associated to the occurrence of tinnitus and/or hyperacusis. However, there are currently no available treatments for cochlear synaptopathy.

Therefore, the present invention aims at providing means for treating hearing diseases such as hearing loss, cochlear synaptopathy and tinnitus, in particular the hearing loss being sensorineural hearing loss.

In the prior art, WO9848802 describes the use of tetrahydropyridine derivatives to prepare medicines for treating diseases causing demyelination such as multiple sclerosis. However, there is nothing in the prior art suggesting that 4-phenyl-tetrahydropyridine derivatives could lead to an improvement of the hearing function.

Yet, the inventors of the present invention have surprisingly discovered that 4-phenyl-tetrahydropyridine derivatives, such as paliroden and xaliproden, lead to an improvement of the hearing function, an improvement of speech intelligibility in silence, an improvement of speech-in-noise intelligibility and exhibit a beneficial effect following noise exposure with auditory brainstem response (ABR) threshold shift reduction.

### SUMMARY

The present invention relates to a compound of formula (I) or a pharmaceutically acceptable salt and/or solvate thereof, for use in the treatment of a hearing disease in a subject, wherein formula (I) is: 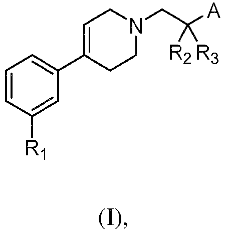 wherein
R₁ is a halogen atom, a CF₃ group, a (C₁-C₄)alkyl group or a (C₁-C₄)alkoxy group;
R₂ and R₃ are independently a hydrogen atom or a (C₁-C₃)alkyl group; and
A is:
   - a phenyl radical substituted by a substituent X, wherein X is:
      (a) a group selected from (C₃-C₆)cycloalkyl, (C₃-C₆)cycloalkylmethyl, (C₃-C₆)cycloalkoxy, (C₃-C₆)cycloalkylamino and cyclohexenyl; or
      (b) a group selected from phenyl, phenylmethyl, phenylcarbonyl, phenoxy, phenylamino, N-(C₁-C₃)alkyl-phenylamino, phenylthio, phenylsulfinyl and phenylsulfonyl; or
   - a 1-naphthyl or 2-naphthyl radical, either non-substituted or substituted in the 5-, 6-, 7- and/or 8-positions by one or two hydroxyl groups, one or two (C₁-C₄)alkoxy groups or a 6,7-methylenedioxy group.

Preferably, R₁ is a CF₃ group.

Preferably, at least one of R₂ and R₃ is a hydrogen atom, more preferably R₂ and R₃ are each a hydrogen atom.

Preferably, A is a biphenyl radical or a non-substituted 2-naphthyl radical.

Preferably, the salt is a hydrochloride salt or a fumarate salt of the compound of formula (I).

Advantageously, said compound is administered to the subject by oral route or by trans-tympanic route. More advantageously, said compound is administered to the subject by oral route or by trans-tympanic route, wherein the administration by trans-tympanic route consists in an injection between the eardrum and the round window by means of a needle which crosses the eardrum. Even more preferably, the compound is administered to the subject by trans-tympanic route, wherein the administration by trans-tympanic route consists in an injection between the eardrum and the round window by means of a needle which crosses the eardrum.

Advantageously, said compound is administered by trans-tympanic route at a dose ranging from 10 µg to 400 mg, between once a month and once every 12 months. Preferably, said administration by trans-tympanic route consists in an injection between the eardrum and the round window by means of a needle which crosses the eardrum.

Preferably, the hearing disease is selected from the group consisting of unilateral or bilateral hearing loss, unilateral or bilateral tinnitus, unilateral or bilateral hyperacusis, hidden hearing loss such as cochlear synaptopathy associated with a normal threshold, speech-intelligibility deficit, unilateral or bilateral temporary auditory threshold shift, central auditory processing disorder, unilateral or bilateral auditory recruitment, acoustic neuroma, single sided deafness, excitotoxicity, ototoxicity such as drug-induced ototoxicity and any combination thereof. More preferably, the hearing disease is selected from the group consisting of unilateral and bilateral hearing loss such as a unilateral and bilateral sensorineural hearing loss, unilateral or bilateral tinnitus, unilateral or bilateral hyperacusis, hidden hearing loss such as cochlear synaptopathy associated with a normal threshold, and speech-intelligibility deficit.

Preferably, the hearing disease is a unilateral or bilateral sensorineural hearing loss selected from the group consisting of unilateral or bilateral noise-induced sensorineural hearing loss, unilateral or bilateral inflammation-induced sensorineural hearing loss, unilateral or bilateral sudden idiopathic sensorineural hearing loss, unilateral or bilateral ototoxic chemicals-induced sensorineural hearing loss and unilateral or bilateral aged-induced sensorineural hearing loss such as presbycusis. More preferably, the hearing disease is a unilateral or bilateral sensorineural hearing loss selected from the group consisting of unilateral or bilateral noise-induced sensorineural hearing loss and unilateral or bilateral aged-induced sensorineural hearing loss.

Preferably, the hearing disease is a unilateral or bilateral inflammation-induced sensorineural hearing loss caused by a chronic inflammatory disease, more preferably said chronic inflammatory disease is selected from the group consisting of diabetes, chronic kidney disease, inflammatory bowel diseases and rheumatoid arthritis.

Preferably, the hearing disease is a unilateral or bilateral hearing loss with a tonal audiometric threshold above 30 dB at 3 contiguous frequencies optionally associated with unilateral or bilateral tinnitus. More preferably, the hearing disease is a unilateral or bilateral hearing loss with a tonal audiometric threshold from 30 dB to 70 dB at 3 contiguous frequencies optionally associated with unilateral or bilateral tinnitus, the tonal audiometric threshold being measured by auditory brainstem response or pure-tone audiometry.

Preferably, the hearing disease is selected from the group consisting of labyrinthine dysfunction, vestibular neuronitis, acute unilateral vestibulopathy, benign paroxysmal positional vertigo, vertigo of central origin, Meniere's disease, Meniere's syndrome and any combination thereof.

In one embodiment, said subject is wearing at least one hearing device.

Advantageously, the compound of formula (I) or a pharmaceutically acceptable salt and/or solvate thereof is administered to the subject during a cochlear surgery, for example during a cochlear implant surgery.

Advantageously, said subject is a mammalian animal with an eardrum, preferably said mammalian animal with an eardrum is selected from the group consisting of a human, a cat, a dog and a non-human primate animal, more preferably said mammalian animal with an eardrum is a dog.

The present invention also relates to a pharmaceutical composition comprising a compound of formula (I) or a pharmaceutically acceptable salt and/or solvate thereof according to the invention, and at least one pharmaceutically acceptable excipient selected from the group consisting of poloxamers, polyethoxylated castor oil, phospholipids, triglycerides and any combination thereof.

The present invention also relates to a pharmaceutical transtympanic composition comprising a compound of formula (I) or a pharmaceutically acceptable salt and/or solvate thereof according to the invention, and a combination of a mixture of polyoxyethylated triglycerides and poloxamers, said pharmaceutical composition being a thermoreversible gel.

The present invention further relates to a pharmaceutical composition for use in the treatment of a hearing disease in a subject, said pharmaceutical composition comprising a compound of formula (I) or a pharmaceutically acceptable salt and/or solvate thereof according to the invention, and at least one pharmaceutically acceptable excipient. Preferably, said hearing disease is as defined in the present description.

### DEFINITIONS

In the present invention, the following terms have the following meanings:
"About", before a figure or number, refers to plus or minus 10% of the face value of that figure or number. In one embodiment, "about", before a figure or number, refers to plus or minus 5% of the face value of that figure or number.
"Active agent" refers to an agent that has a therapeutic effect. The agent may be a chemical or a biological substance. Preferably, the active agent is a chemical substance. The therapeutic effect may be the prevention, delay, reduction in severity and/or frequency or suppression of at least one symptom associated with a pathological condition, or the prevention, slowing down or suppression of the underlying cause of a pathological condition, or the improvement or repair of a damage.
"Alkyl" by itself or as part of another substituent refers to a linear saturated hydrocarbyl group. Non-limiting examples of alkyl groups include methyl, ethyl, n-propyl, iso-propyl, n-butyl, iso-butyl, sec-butyl, tert-butyl, n-pentyl, iso-pentyl, tert-pentyl, n-hexyl, iso-hexyl and tert-hexyl groups. Preferred alkyl groups include methyl, ethyl, n-propyl, iso-propyl, n-butyl, sec-butyl and tert-butyl groups. "**Cycloalkyl**" by itself or as part of another substituent refers to a cyclic saturated hydrocarbyl group. "**Alkoxy**" refers to -O-alkyl. "**Cycloalkoxy**" refers to -O-cycloalkyl. "**Cycloalkylamino**" refers to -NH-cycloalkyl.
"**Audiometric threshold**" or "**tonal audiometric threshold**" or "**hearing threshold**" or "**threshold**" refers to the sound level below which a person's ear is unable to detect any sound, i.e. "threshold" is the hearing level at which the test stimulus is just barely audible. The audiometric threshold may be measured, for example, objectively by auditory brainstem response or subjectively by pure-tone audiometry. "**ABR threshold**" refers to a threshold measured by auditory brainstem response. "**Pure-tone audiometric threshold**" refers to a threshold measured by pure-tone audiometry. A "**normal threshold**" is from -10 to 15 dB. An "**increased threshold**" refers to a threshold greater than or equal to 16 dB.
"**Auditory brainstem response**" or "**ABR**" refers to an auditory evoked potential extracted from ongoing electrical activity in the brain and recorded via electrodes placed on the scalp.
"**Buffer**" relates to a mixture of a weak acid and its conjugate base, or a weak base and its conjugate acid, allowing the pH of a pharmaceutical composition to be maintained constant when a small amount of strong acid or base is added thereto. In an embodiment, the buffer is a phosphate buffer. In an embodiment, the buffer is a bicarbonate buffer. A "**phosphate buffer**" is a buffer which comprises phosphate or a derivative thereof as buffering agent. A "**bicarbonate buffer**" is a buffer which comprises bicarbonate or a derivative thereof as buffering agent.
"**Buffering agent**" refers to the specific chemical that is present in both an acidic and a basic forms in a buffer, allowing the pH of a pharmaceutical composition to be maintained constant. In an embodiment, the buffering agent is phosphate or a derivative thereof. In another embodiment, the buffering agent is bicarbonate or a derivative thereof.
"**Chronic disease**" refers to a long-term, progressive illness, often associated with disability and the threat of serious complications. Chronic diseases evolve more or less rapidly for at least several months, in particular at least 3 months.
"**Chronic inflammatory disease**" refers to a chronic disease which comprises, among its causes and/or symptoms and/or consequences, a chronic inflammatory reaction affecting one or several organs or tissues, said chronic inflammatory reaction lasting at least 3 months. In one embodiment, the chronic inflammatory disease is selected from the group consisting of rheumatoid arthritis, chronic inflammatory bowel diseases (IBD) (consisting of Crohn's disease (CD) and ulcerative colitis (UC)), type 1 diabetes, type 2 diabetes, chronic kidney disease (CKD), psoriasis, psoriatic arthritis, ulcerative colitis, ankylosing spondylitis, inflammatory myopathies, systemic lupus erythematosus, Gougerot-Sjögren syndrome, scleroderma and thyroiditis. In a preferred embodiment, the chronic inflammatory disease is selected from the group consisting of rheumatoid arthritis, Crohn's disease, ulcerative colitis, type 1 diabetes, type 2 diabetes and chronic kidney disease. In a more preferred embodiment, the chronic inflammatory disease is type 2 diabetes.
"**Cochlear hair cells**" refers to hair cells distributed in a tonotopic organization along the cochlea. They encode a wide range of sound frequencies with high- to low-frequency sounds being coded from the base to apex of the cochlea respectively;; they include inner hair cells and outer hair cells. The "**inner hair cells**" (IHC) encode sound waves into electric signals transmitted via the auditory nerve to the cochlear nucleus in the brainstem, then transmitted through the central auditory system, to finally reach the auditory cortex; they are essential for hearing and encode a wide range of sound intensities through a gradual recruitment of different type I afferent nerve fibers, ranging from high spontaneous rate and low threshold fibers to encode low intensity sounds and up to low spontaneous rate and high thresholds nerve fibers to encode high intensity sounds. The "**outer hair cells**" (OHC), which connect to type II afferent nerve fibers as well as efferent nerve fibers, amplify low intensity sounds through adaptative contraction activities and are therefore responsible for hearing sensitivity; the OHC are more sensitive than IHC to damage induced by excessive noise levels and/or by ototoxic chemicals.
"**Cochlear synaptopathy**" refers to a hearing disease. It is due to a decrease in the number of synapses between IHC and spiral ganglion neurons (SGN) nerve fibers. The etiology of cochlear synaptopathy may be the exposition to excessive noise levels, said exposition causing a disruption of synaptic connections between inner hair cells and auditory nerve fibers of SGNs by excessive release of glutamate (known as excitotoxicity), and then to the death and gradual disappearance of disconnected SGNs. A cochlear synaptopathy is characterized by an alteration in speech intelligibility; the patients with a cochlear synaptopathy have difficulty processing speech information correctly in a background noise context, i.e. they have a speech intelligibility deficit in noise. Advantageously, a subject with a cochlear synaptopathy may be a human aged from 40 to 65 years.
   A cochlear synaptopathy may be either a hidden form of hearing loss (i.e. a cochlear synaptopathy associated with a normal threshold) or associated with a hearing loss (i.e. a cochlear synaptopathy associated with an increased threshold).
   Patients with a "**cochlear synaptopathy associated with a normal threshold**" have a normal hearing threshold. A cochlear synaptopathy associated with a normal threshold is a hidden form of hearing loss, also named a sub-clinical form of hearing loss. A cochlear synaptopathy associated with a normal threshold is a hearing disease characterized by an alteration in speech intelligibility while the hearing ability is maintained with a normal threshold. Thus, patients with a cochlear synaptopathy associated with a normal threshold have difficulty processing speech information correctly in a background noise context, i.e. they have a speech intelligibility deficit in noise, while having a normal hearing threshold.
   Patients with a "**cochlear synaptopathy associated with an increased threshold**" have an increased hearing threshold, in particular a hearing threshold from 16 to 40 dB. A cochlear synaptopathy associated with an increased threshold is a hearing loss associated with an alteration in speech intelligibility. The patients with a cochlear synaptopathy associated with an increased threshold have difficulty processing speech information correctly in a background noise context, i.e. they have a speech intelligibility deficit in noise, and they have an increased hearing threshold.
"**Comprising**" or "**comprise**" is to be construed in an open, inclusive sense, but not limited to. In an embodiment, "comprising" means "consisting essentially of". In an embodiment, "comprising" means "consisting of", which is to be construed as limited to.
"**Three contiguous frequencies**" refers to 3 contiguous frequencies selected among the following frequencies: 0.25 kHz, 0.5 kHz, 1 kHz, 2 kHz, 3 kHz, 4 kHz, 6 kHz, 8 kHz, 12 kHz and 16 kHz. Preferably, the 3 contiguous frequencies are selected among the following frequencies: 0.25 kHz, 0.5 kHz, 1 kHz, 2 kHz, 3 kHz, 4 kHz, 6 kHz, and 8 kHz. For example, the 3 contiguous frequencies may be selected from the group consisting of:
   - 0.5 kHz, 1 kHz and 2 kHz (corresponding to the speech frequencies range);
   - 1 kHz, 2 kHz and 4 kHz (corresponding to the middle frequencies range);
   - 4 kHz, 6 kHz and 8kHz (corresponding to the high frequencies range); and
   - 8 kHz, 12 kHz and 16kHz (corresponding to the extended high frequencies range).
"**dB SPL**" refers to decibels relative to the sound pressure threshold of human hearing. "**dB SPL RMS**" refers to a measurement of the average dB SPL. SPL = sound pressure levels. RMS = root mean square.
"**Dose**" refers to the amount of active agent administered at one time. In one embodiment, two oral doses are administered to one subject from 8 hours to 1 week apart, preferably from 12 hours to 24 hours apart, more preferably about 24 hours apart. In one embodiment, two trans-tympanic doses are administered to one subject from 1 week to 12 months apart, for example 1 week apart, 12 weeks apart, 1 month apart, 3 months apart, 6 months apart or 12 months apart; preferably, two trans-tympanic doses are administered to one subject 1 month apart. Advantageously, a human dose is a standard human dose for a man weighing 70 kg.
"**Eardrum**" refers to a membrane that separates the external ear from the middle ear.
"**Excipient**" refers to any inactive ingredient, which is required for the formulation of an active agent in a suitable dosage form. In one embodiment, "excipient" refers to any and all solvents, diluents carriers, fillers, bulking agents, binders, disintegrants, polymer, lubricant, glidant, surfactants, isotonic agents, thickening or emulsifying agents, stabilizers, absorption accelerators, flavoring agents, preservatives, antioxidants, buffering agents, gelling agent, solubilizing agent or any combination thereof. Excipients are, by definition, inactive; however, just in case, it is specified that the term "excipient" according to the invention refers to non-ototoxic excipients.
"**From X to Y**" refers to the range of values between X and Y, the limits X and Y being included in said range.
"**Gel**" refers to a three-dimensional network of solid elements (forming a matrix) diluted in a liquid (that acts as a blowing agent), the gel exhibiting no flow when in a stable state and having properties ranging from soft and ductile to hard and brittle. "**Thermoreversible gel**" refers to a gel that has a melting temperature over which the gel is in a viscous fluid state and flows, and below which the gel is in the gel state, the junction points of said thermoreversible gel being thermally reversible.
"**Halogen**" refers to fluorine, chlorine, bromine or iodine.
"**Hearing disease**" refers to a disease that affects the audition or the ear (for example an ear infection such as an external otitis). In one embodiment, "hearing disease" refers to a disease that affects the audition. A disease that affects the audition may affect the hearing threshold or the speech intelligibility such as the speech intelligibility in noise.
"**Hearing loss**" refers to a hearing disease characterized by an increase of hearing threshold, greater than or equal to 16 dB. A hearing loss may be temporary or permanent. A slight hearing loss is characterized by a threshold from 16 to 25 dB. A mild hearing loss is characterized by a threshold from 26 to 40 dB. A moderate hearing loss is characterized by a threshold from 41 to 55 dB. A moderately severe hearing loss is characterized by a threshold from 56 to 70 dB. A severe hearing loss is characterized by a threshold from 71 to 90 dB. A profound hearing loss is characterized by a threshold greater than or equal to 91 dB.
"**Hidden form of hearing loss**" or "**sub-clinical form of hearing loss**" refers to a hearing disease characterized by an alteration in speech intelligibility while the hearing ability is maintained with a normal threshold. Thus, patients with a hidden form of hearing loss, such as a cochlear synaptopathy associated with a normal threshold, have difficulty processing speech information correctly in a background noise context, i.e. they have a speech intelligibility deficit in noise, while having a normal hearing threshold.
"**Lipid-based solution**" refers to a solution in which the solvent comprises at least one lipid. Preferably, the solvent comprises at least 20%, more preferably at least 40%, even more preferably at least 50% by weight of at least one lipid, in relation to the total weight of the solvent.
"**Mucoadhesive**" refers to a substance or composition adhering to a mucous membrane of the ear. In one embodiment, "mucoadhesive" refers to a substance or composition adhering to the mucous membrane of the middle ear.
"**Ototoxic chemicals**" refers to chemicals that can induce a hearing loss in a subject when absorbed into the bloodstream of said subject. The ototoxic chemicals may be solvents (e.g. butanol, carbon disulfide, ethanol), heavy metals (e.g. arsenic, lead, manganese), asphyxiants (e.g. acrylonitrile, carbon monoxide, hydrogen cyanide) or medications (e.g. platinum based antineoplastic drugs, aminoglycosides). In one embodiment, "ototoxic chemicals" refers to ototoxic medications, such as platinum based antineoplastic drugs or aminoglycosides.
"**Paliroden**" refers to the chemical molecule 1-[2-(4-biphenylyl)ethyl]-4-(3-trifluoromethylphenyl)-1,2,3,6-tetrahydropyridine, of the following chemical formula: 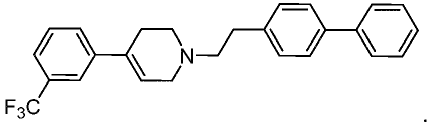
**"Pharmaceutical composition"** refers to the combination of at least one active agent and at least one pharmaceutically acceptable excipient.
**"Pharmaceutically acceptable"** refers to generally safe, non-toxic and neither biologically nor physiologically nor otherwise undesirable for mammalian animals, in particular for humans, dogs, cats and non-human primate animals.
**"Phenylcarbonyl"** refers to -CO-phenyl. "**Phenoxy**" refers to -O-phenyl. **"Phenylamino"** refers to -NH-phenyl. **"Phenylthio"** refers to -S-phenyl. **"Phenylsulfinyl"** refers to -SO-phenyl. **"Phenylsulfonyl"** refers to -SO₂-phenyl.
"**Placebo**" or "**vehicle**" refers to a pharmaceutical composition that comprises only pharmaceutically acceptable excipient(s), and no active agent.
"**Poloxamer**" refers to polyethylene glycol - polypropylene glycol - polyethylene glycol block terpolymers.
"**Poloxamer 188**" is a poloxamer having an average molecular weight of 8,400 g/mol.
"**Poloxamer 407**" is a poloxamer having an average molecular weight of 12,600 g/mol, an approximate length of the two PEG blocks of 101 repeat units and an approximate length of the propylene glycol block of 56 repeat units.
"**Pure-tone audiometry**" refers to the measurement of an individual's hearing sensitivity for calibrated pure tones.
"**Residual hearing**" refers to the physiological hearing ability in a patient with a hearing loss, thus without taking into account the eventual hearing gain induced by hearing aids, if the patient has hearing aids such as cochlear implants.
"**Salt of a compound**" refers to acid or base addition salts of said compound. The acid addition salts are formed with pharmaceutically acceptable organic or inorganic acids; the base addition salts are formed when an acid proton present in the compound is either replaced by a metal ion or coordinated with a pharmaceutically acceptable organic or inorganic base. In one embodiment, the acid addition salt is selected from the group consisting of acetate, adipate, aspartate, benzoate, besylate, bicarbonate/carbonate, bisulphate/sulphate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts. In one embodiment, the base addition salt is selected from the group consisting of aluminium, arginine, benzathine, calcium, choline, diethylamine, 2-(diethylamino)ethanol, diolamine, ethanolamine, glycine, 4-(2-hydroxyethyl)-morpholine, lysine, magnesium, meglumine, morpholine, olamine, potassium, sodium, tromethamine and zinc salts. According to a preferred embodiment, the salt is selected from the group consisting of besylate, fumarate, and hydrochloride/chloride salts.
"**Sensorineural hearing loss**" or "**SNHL**" refers to the most common type of hearing disease in adults, due to a loss of sensory hair cells function in the cochlea. The etiology of SNHL may be the exposition to excessive noise levels and/or to ototoxic chemicals such as platinum based antineoplastic drugs and aminoglycosides, said exposition causing damage to inner and outer hair cells and leading to transient or permanent hearing loss.
"**Solvate of a compound**" refers to a molecular complex comprising said compound and one or more pharmaceutically acceptable solvent molecules. "**Hydrate of a compound**" refers to a molecular complex comprising the compound and one or more pharmaceutically acceptable solvent molecules, wherein the solvent is water.
"**Subject**" or "**patient**" refers to a mammalian animal, wherein "**mammalian animal**" refers to a human or a non-human mammalian animal. Preferably, "subject" refers to a mammalian animal with an eardrum. In one embodiment, "subject" refers to a human (man or woman). According to a preferred embodiment, "subject" refers to a human over the age of 18, preferably over the age of 40, more preferably over the age of 50, even more preferably over the age of 65. In particular, "subject" may refer to a human aged from 40 to 65 years. In another embodiment, "subject" refers to a non-human mammalian animal, preferably to a non-human mammalian animal selected from the group consisting of a cat, a dog, a horse and a non-human primate such as a monkey. According to a preferred embodiment, "subject" refers to a non-human mammalian animal selected from the group consisting of a cat and a dog, more preferably "subject" refers to a non-human mammalian animal, wherein said non-human mammalian animal is a dog.
"**Sustained-release composition**" refers to a composition that allows the release of its active agent(s) over an extended period of time, preferably for at least 12 hours, more preferably for at least 24 hours, even more preferably for at least one week.
"**Therapeutically effective amount**" or "**effective amount**" of an active agent or of a composition refers to a nontoxic but sufficient amount of said active agent or composition to provide the desired therapeutic effect.
"**Tinnitus**" refers to a hearing disease in which the patient hears phantom noises intermittently or continuously, said noises being not caused by sounds coming from the outside world and thus said noises being not heard by other people than said patient. Such noises may be ringing, buzzing, humming, hissing, throbbing, music or singing. Tinnitus is a common problem, which affects about 15% of people..
"**Trans-tympanic administration**" or "**intra-tympanic administration**" or "**administration by trans-tympanic route**" refers to the administration of an active agent or a composition that crosses the eardrum from the external ear canal.

In one embodiment, the trans-tympanic administration consists in the administration of an active agent or a composition in the external ear canal, wherein said active agent or the active agent of said composition is able to cross the eardrum, for example by diffusion. The formulation of the active agent or of the composition may be any formulation suitable for administration in the external ear canal. Preferably, the formulation of the active agent or of the composition is selected from the group consisting of a semi-solid formulation, gel formulation and liquid formulation. The liquid formulation may be selected from the group consisting of a suspension, an emulsion and a solution. The gel formulation may be selected from the group consisting of a thermoreversible gel, hydrogel, glycerin-based gel, conjugated gal, crosslinked gel and alginate-based gel. The formulation of the active agent or of the composition may be an osmotic dosage formulation, a diffusion dosage formulation or an erodible formulation. More preferably, the formulation of the active agent or of the composition is selected from the group consisting of a gel formulation and a liquid formulation, for example an ear gel or ear drops.

In another embodiment, the trans-tympanic administration consists in an injection between the eardrum and the round window by means of a needle which crosses the eardrum. After the trans-tympanic injection, the injected substance diffuses through the round window to the cochlea. Preferably, the gauge of the needle is from 21 to 33, more preferably from 21 to 30, more preferably from 24 to 30, more preferably from 25 to 30, even more preferably from 25 to 27, even more preferably from 25 to 26. In particular, the gauge of the needle may be selected from the group consisting of 21, 22, 23, 24, 25, 26, 27, 28, 29, 30, 31, 32 and 33. More preferably, the needle is a 26 gauge-needle (=26G needle). Advantageously, the needle is selected from the group consisting of a trans-tympanic injection needle and a spinal needle. More advantageously, the needle is a trans-tympanic injection needle. For example, the needle may be the product "MediPlast ENT, Otology, Trans- Intratympanic Injection Needle, Single Use With Luer-lock 0.4 x 90mm" marketed by company Mediplast^{®}.
Preferably, the formulation of the active agent or of the composition is selected from the group consisting of a semi-solid formulation, gel formulation and liquid formulation. The liquid formulation may be selected from the group consisting of a suspension, an emulsion and a solution. The solution may be a mucoadhesive solution, in particular a lipid-based mucoadhesive solution. The gel formulation may be selected from the group consisting of a thermoreversible gel, hydrogel, glycerin-based gel, conjugated gal, crosslinked gel and alginate-based gel. The formulation of the active agent or of the composition may be an osmotic dosage formulation, a diffusion dosage formulation or an erodible formulation.
More preferably, the formulation of the active agent or of the composition is selected from the group consisting of a gel formulation and a liquid formulation. Even more preferably, the formulation of the active agent or of the composition is selected from the group consisting of a thermoreversible gel and a solution; the solution is in particular a mucoadhesive solution, more particularly a lipid-based mucoadhesive solution.

"**Treating**" or "**treatment**" refers to any action which makes it possible to prevent, delay, reduce in severity and/or frequency or suppress at least one symptom associated with a pathological condition, or to prevent, slow down or suppress the underlying cause of a pathological condition, or the improvement or remediation of damage. In one embodiment, **"treatment"** refers to a curative treatment. In another embodiment, **"treatment"** refers to a preventive treatment. In another embodiment, **"treatment"** refers to a preventive and/or curative treatment.

**"Unilateral or bilateral"** refers to a hearing disease that may affect either one ear (this is a unilateral hearing disease) or both ears (this is a bilateral hearing disease). A unilateral hearing disease may affect either the right ear or the left ear.

**"Xaliproden"** refers to the chemical molecule 1-[2-(2-naphthyl)ethyl]-4-(3-trifluoromethylphenyl)-1,2,3,6-tetrahydropyridine, of the following chemical formula:

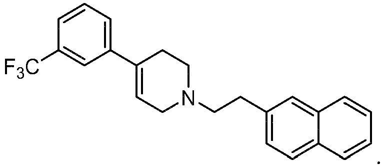

### DETAILED DESCRIPTION

### Compound for use

### Formula (I)

The present invention relates to a compound of formula (I) or a pharmaceutically acceptable salt and/or solvate thereof, for use in the treatment of a hearing disease in a subject.

The present invention also relates to a method of treating a hearing disease by administering to a subject in need thereof an effective amount of a compound of formula (I) or a pharmaceutically acceptable salt and/or solvate thereof.

Any references to methods of treatment in this description are to be interpreted as references to the compounds, pharmaceutical compositions or medicaments of the present invention for use in those.

The present invention also relates to the use of a compound of formula (I) or a pharmaceutically acceptable salt and/or solvate thereof for the treatment of a hearing disease in a subject.

Formula (I) is: 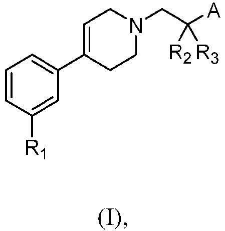 wherein
R₁ is a halogen atom, a CF₃ group, a (C₁-C₄)alkyl group or a (C₁-C₄)alkoxy group;
R₂ and R₃ are independently a hydrogen atom or a (C₁-C₃)alkyl group; and
A is:
   - a phenyl radical substituted by a substituent X, wherein X is:
      (a) a group selected from (C₃-C₆)cycloalkyl, (C₃-C₆)cycloalkylmethyl, (C₃-C₆)cycloalkoxy, (C₃-C₆)cycloalkylamino and cyclohexenyl; or
      (b) a group selected from phenyl, phenylmethyl, phenylcarbonyl, phenoxy, phenylamino, N-(C₁-C₃)alkyl-phenylamino, phenylthio, phenylsulfinyl and phenylsulfonyl; or
   - a 1-naphthyl or 2-naphthyl radical, either non-substituted or substituted in the 5-, 6-, 7- and/or 8-positions by one or two hydroxyl groups, one or two (C₁-C₄)alkoxy groups or a 6,7-methylenedioxy group.

Preferably, R₁ is a CF₃ group.

Preferably, at least one of R₂ and R₃ is a hydrogen atom. More preferably, R₂ and R₃ are each a hydrogen atom.

Preferably, A is a biphenyl radical or a non-substituted 2-naphthyl radical.

According to one embodiment, in the above formula (I):
R₁ is a halogen atom, a CF₃ group, or a (C₁-C₄)alkoxy group;
R₂ and R₃ are independently a hydrogen atom or a (C₁-C₃)alkyl group; and
A is:
   - a phenyl radical substituted by a substituent X, wherein X is a group selected from phenyl, phenylmethyl, phenylcarbonyl, phenoxy, phenylamino, N-(C₁-C₃)alkyl-phenylamino, phenylthio, phenylsulfinyl and phenylsulfonyl; or
   - a 1-naphthyl or 2-naphthyl radical, either non-substituted or substituted in the 5-, 6-, 7- and/or 8-positions by one or two hydroxyl groups, one or two (C₁-C₄)alkoxy groups or a 6,7-methylenedioxy group.

According to one embodiment, in the above formula (I):
R₁ is a halogen atom, a CF₃ group, or a (C₁-C₄)alkoxy group;
R₂ and R₃ are independently a hydrogen atom or a (C₁-C₃)alkyl group; and
A is:
   - a biphenyl; or
   - a non-substituted 1-naphthyl or 2-naphthyl radical, preferably a non-substituted 2-naphthyl radical.

According to one embodiment, in the above formula (I):
R₁ is a halogen atom, a CF₃ group, or a (C₁-C₄)alkoxy group;
R₂ and R₃ are independently a hydrogen atom or a (C₁-C₃)alkyl group; and
A is a biphenyl.

According to one embodiment, in the above formula (I):
R₁ is a halogen atom, a CF₃ group, or a (C₁-C₄)alkoxy group;
R₂ and R₃ are independently a hydrogen atom or a (C₁-C₃)alkyl group; and
A is a non-substituted 2-naphthyl radical.

According to a preferred embodiment, formula (I) is: 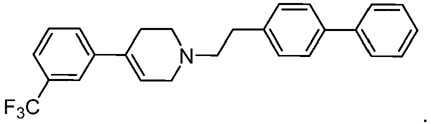 According to this embodiment, the compound of formula (I) or a pharmaceutically acceptable salt and/or solvate thereof is 1-[2-(4-biphenylyl)ethyl]-4-(3-trifluoromethylphenyl)-1,2,3,6-tetrahydropyridine, also named paliroden, or a pharmaceutically acceptable salt and/or solvate thereof.

According to another preferred embodiment, formula (I) is: 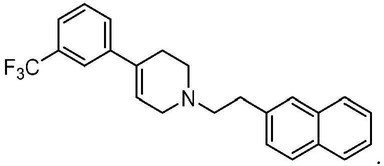 According to this embodiment, the compound of formula (I) or a pharmaceutically acceptable salt and/or solvate thereof is 1-[2-(2-naphthyl)ethyl]-4-(3-trifluoromethylphenyl)-1,2,3,6-tetrahydropyridine, also named xaliproden, or a pharmaceutically acceptable salt and/or solvate thereof.

Advantageously, the salt is selected from the group consisting of a hydrochloride salt, a fumarate salt and a besylate salt of the compound of formula (I). More advantageously, the salt is selected from the group consisting of a hydrochloride salt and a fumarate salt of the compound of formula (I).

According to a preferred embodiment, the compound of formula (I) or a pharmaceutically acceptable salt and/or solvate thereof is a fumarate salt of 1-[2-(4-biphenylyl)ethyl]-4-(3-trifluoromethylphenyl)-1,2,3,6-tetrahydropyridine, also named paliroden fumarate.

According to another preferred embodiment, the compound of formula (I) or a pharmaceutically acceptable salt and/or solvate thereof is a hydrochloride salt of 1-[2-(2-naphthyl)ethyl]-4-(3-trifluoromethylphenyl)-1,2,3,6-tetrahydropyridine, also named xaliproden hydrochloride.

### Indications

According to the present invention, the compound of formula (I) or a pharmaceutically acceptable salt and/or solvate thereof as defined above is for use in the treatment of a hearing disease in a subject.

According to one embodiment, the hearing disease is selected from the group consisting of unilateral or bilateral hearing loss, unilateral or bilateral tinnitus, hidden hearing loss such as cochlear synaptopathy associated with a normal threshold, otosclerosis such as non-obliterative and obliterative otosclerosis involving oval window and cochlear otosclerosis, acoustic neuroma, single sided deafness, ototoxicity such as drug-induced ototoxicity, labyrinthine dysfunction, vestibular neuronitis, excitotoxicity, autoimmune inner ear disease, acute unilateral vestibulopathy, benign paroxysmal positional vertigo, vertigo of central origin, Meniere's disease, Meniere's syndrome, unilateral or bilateral auditory recruitment, diplacusis, unilateral or bilateral hyperacusis, unilateral or bilateral temporary auditory threshold shift, central auditory processing disorder and any combination thereof.

Advantageously, the unilateral or bilateral hearing loss is selected from the group consisting of unilateral or bilateral sensorineural hearing loss, unilateral or bilateral cochlear synaptopathy associated with an increased threshold, and unilateral or bilateral mixed conductive and sensorineural hearing loss. Preferably, the unilateral or bilateral sensorineural hearing loss is selected from the group consisting of unilateral or bilateral noise-induced sensorineural hearing loss, unilateral or bilateral inflammation-induced sensorineural hearing loss, unilateral or bilateral sudden idiopathic sensorineural hearing loss, unilateral or bilateral ototoxic chemicals-induced sensorineural hearing loss and unilateral or bilateral aged-induced sensorineural hearing loss such as presbycusis. More preferably, the unilateral or bilateral sensorineural hearing loss is selected from the group consisting of unilateral or bilateral noise-induced sensorineural hearing loss, unilateral or bilateral aged-induced sensorineural hearing loss such as presbycusis, and unilateral or bilateral inflammation-induced sensorineural hearing loss. The unilateral or bilateral inflammation-induced sensorineural hearing loss may be induced by a chronic inflammatory disease. Even more preferably, the unilateral or bilateral sensorineural hearing loss is selected from the group consisting of unilateral or bilateral noise-induced sensorineural hearing loss and unilateral or bilateral aged-induced sensorineural hearing loss such as presbycusis. Preferably, the unilateral or bilateral cochlear synaptopathy associated with an increased threshold is selected from the group consisting of unilateral or bilateral noise-induced cochlear synaptopathy associated with an increased threshold, unilateral or bilateral inflammation-induced cochlear synaptopathy associated with an increased threshold, and unilateral or bilateral aged-induced cochlear synaptopathy associated with an increased threshold. In particular, the unilateral or bilateral noise-induced cochlear synaptopathy associated with an increased threshold may be induced by chronic noise exposure. In particular, the unilateral or bilateral inflammation-induced cochlear synaptopathy associated with an increased threshold may be induced by a chronic inflammatory disease.

According to a preferred embodiment, the hearing disease is selected from the group consisting of unilateral and bilateral sensorineural hearing loss, unilateral and bilateral cochlear synaptopathy associated with an increased threshold, unilateral or bilateral tinnitus, cochlear synaptopathy associated with a normal threshold, acoustic neuroma, sudden idiopathic hearing loss, drug-induced hearing loss, single sided deafness, excitotoxicity, ototoxicity, drug-induced ototoxicity, labyrinthine dysfunction, vestibular neuronitis, acute unilateral vestibulopathy, benign paroxysmal positional vertigo, vertigo of central origin, Meniere's disease and Meniere's syndrome.

According to a preferred embodiment, the hearing disease is selected from the group consisting of unilateral or bilateral hearing loss, unilateral or bilateral tinnitus, hidden hearing loss such as cochlear synaptopathy associated with a normal threshold, otosclerosis such as non-obliterative and obliterative otosclerosis involving oval window and cochlear otosclerosis, acoustic neuroma, single sided deafness, ear infection, ototoxicity such as drug-induced ototoxicity, excitotoxicity, acute unilateral vestibulopathy, vestibular neuronitis and any combination thereof.

According to a more preferred embodiment, the hearing disease is selected from the group consisting of unilateral or bilateral hearing loss, unilateral or bilateral tinnitus, hidden hearing loss such as cochlear synaptopathy associated with a normal threshold, single sided deafness, acute unilateral vestibulopathy, vestibular neuronitis, unilateral or bilateral auditory recruitment, diplacusis, unilateral or bilateral hyperacusis, unilateral or bilateral temporary auditory threshold shift, central auditory processing disorder and any combination thereof.

According to a more preferred embodiment, the hearing disease is selected from the group consisting of unilateral or bilateral tinnitus, unilateral or bilateral auditory recruitment, diplacusis, unilateral or bilateral hyperacusis, unilateral or bilateral temporary auditory threshold shift, central auditory processing disorder and any combination thereof. These hearing diseases correspond to those of the classes H93.1 and H93.2 of the International Classification of Diseases ICD-10, version 2019.

According to a more preferred embodiment, the hearing disease is selected from the group consisting of unilateral or bilateral hearing loss, unilateral or bilateral tinnitus, hidden hearing loss such as cochlear synaptopathy associated with a normal threshold and any combination thereof.

According to a more preferred embodiment, the hearing disease is selected from the group consisting of unilateral or bilateral hearing loss, unilateral or bilateral tinnitus, hidden hearing loss such as cochlear synaptopathy associated with a normal threshold and any combination thereof, wherein the unilateral or bilateral hearing loss is selected from the group consisting of unilateral or bilateral sensorineural hearing loss, unilateral or bilateral mixed conductive and sensorineural hearing loss, and unilateral or bilateral cochlear synaptopathy associated with an increased threshold. Preferably, the hearing disease is a unilateral or bilateral hearing loss, wherein the unilateral or bilateral hearing loss is selected from the group consisting of 1) a unilateral or bilateral sensorineural hearing loss, such as a noise-induced sensorineural hearing loss, or an aged-induced sensorineural hearing loss such as presbycusis, and 2) a unilateral or bilateral cochlear synaptopathy associated with an increased threshold, such as a noise-induced cochlear synaptopathy associated with an increased threshold or an aged-induced cochlear synaptopathy associated with an increased threshold.

According to a more preferred embodiment, the hearing disease is selected from the group consisting of unilateral or bilateral sensorineural hearing loss such as noise-induced sensorineural hearing loss or aged-induced sensorineural hearing loss such as presbycusis, unilateral or bilateral cochlear synaptopathy associated with an increased threshold, unilateral or bilateral tinnitus, hidden hearing loss such as cochlear synaptopathy associated with a normal threshold and any combination thereof.

According to a more preferred embodiment, the hearing disease is selected from the group consisting of unilateral or bilateral sensorineural hearing loss such as noise-induced sensorineural hearing loss or aged-induced sensorineural hearing loss such as presbycusis, unilateral or bilateral tinnitus and any combination thereof.

According to a more preferred embodiment, the hearing disease is a combination of a unilateral or bilateral sensorineural hearing loss, such as noise-induced sensorineural hearing loss or aged-induced sensorineural hearing loss, and unilateral or bilateral tinnitus.

According to a more preferred embodiment, the hearing disease is a combination of unilateral or bilateral tinnitus and a hidden hearing loss such as cochlear synaptopathy associated with a normal threshold.

According to a more preferred embodiment, the hearing disease is a combination of a unilateral or bilateral sensorineural hearing loss, such as noise-induced sensorineural hearing loss or aged-induced sensorineural hearing loss, and a hidden hearing loss such as cochlear synaptopathy associated with a normal threshold.

According to a more preferred embodiment, the hearing disease is a combination of a unilateral or bilateral sensorineural hearing loss, such as noise-induced sensorineural hearing loss or aged-induced sensorineural hearing loss, and a unilateral or bilateral cochlear synaptopathy associated with an increased threshold.

According to a more preferred embodiment, the hearing disease is a combination of a unilateral or bilateral tinnitus, and a unilateral or bilateral cochlear synaptopathy associated with an increased threshold.

According to a more preferred embodiment, the hearing disease is a unilateral or bilateral sensorineural hearing loss, preferably the unilateral or bilateral sensorineural hearing loss is selected from a noise-induced sensorineural hearing loss and an aged-induced sensorineural hearing loss such as presbycusis.

According to a more preferred embodiment, the hearing disease is unilateral or bilateral tinnitus.

According to a more preferred embodiment, the hearing disease is a hidden hearing loss such as cochlear synaptopathy associated with a normal threshold.

According to a more preferred embodiment, the hearing disease is a unilateral or bilateral cochlear synaptopathy associated with an increased threshold.

Preferably, the hearing disease according to the invention is not induced by a decrease or an increase in TGF-β (Transforming Growth Factor β).

Preferably, the hearing disease according to the invention is not induced by a demyelinating disease nor by a demyelination of the auditory nerve.

In one embodiment, the hearing disease according to the invention is an inflammation-induced unilateral or bilateral sensorineural hearing loss caused by a chronic inflammatory disease, preferably said chronic inflammatory disease is selected from the group consisting of diabetes preferably type 2 diabetes, chronic kidney disease, inflammatory bowel diseases and rheumatoid arthritis.

In one embodiment, the hearing disease according to the invention is an inflammation-induced unilateral or bilateral cochlear synaptopathy associated with an increased threshold caused by a chronic inflammatory disease, preferably said chronic inflammatory disease is selected from the group consisting of diabetes preferably type 2 diabetes, chronic kidney disease, inflammatory bowel diseases and rheumatoid arthritis.

In another embodiment, the hearing disease according to the invention is a sensorineural hearing loss that is not caused by diabetes, chronic kidney disease, inflammatory bowel diseases or rheumatoid arthritis, preferably the hearing disease according to the invention is a sensorineural hearing loss that is not caused by a chronic inflammatory disease.

In another embodiment, the hearing disease according to the invention is a unilateral or bilateral cochlear synaptopathy associated with an increased threshold that is not caused by diabetes, chronic kidney disease, inflammatory bowel diseases or rheumatoid arthritis, preferably the hearing disease according to the invention is a unilateral or bilateral cochlear synaptopathy associated with an increased threshold that is not caused by a chronic inflammatory disease.

According to an embodiment, the hearing disease is a unilateral or bilateral hearing loss with a tonal audiometric threshold above 30 dB at 3 contiguous frequencies, optionally associated with unilateral or bilateral tinnitus, the tonal audiometric threshold being measured by auditory brainstem response or pure-tone audiometry. According to a preferred embodiment, the hearing disease is a unilateral or bilateral hearing loss with a tonal audiometric threshold from 30 dB to 70 dB at 3 contiguous frequencies, optionally associated with unilateral or bilateral tinnitus, the tonal audiometric threshold being measured by auditory brainstem response or pure-tone audiometry. According to another embodiment, the hearing disease is a unilateral or bilateral hearing loss with a tonal audiometric threshold greater than or equal to 71 dB at 3 contiguous frequencies, optionally associated with unilateral or bilateral tinnitus, the tonal audiometric threshold being measured by auditory brainstem response or pure-tone audiometry. According to preferred embodiment, the hearing disease is a bilateral hearing loss with a tonal audiometric threshold greater than or equal to 71 dB at 3 contiguous frequencies, the tonal audiometric threshold being measured by auditory brainstem response or pure-tone audiometry. According to another embodiment, the hearing disease is a unilateral or bilateral hearing loss with a tonal audiometric threshold greater than 91 dB at 3 contiguous frequencies, optionally associated with unilateral or bilateral tinnitus, the tonal audiometric threshold being measured by auditory brainstem response or pure-tone audiometry. According to preferred embodiment, the hearing disease is a unilateral hearing loss with a tonal audiometric threshold greater than or equal to 91 dB at 3 contiguous frequencies, associated with unilateral or bilateral tinnitus, the tonal audiometric threshold being measured by auditory brainstem response or pure-tone audiometry.

Advantageously, the subject is wearing at least one hearing device. The subject may be wearing one hearing device. The subject may also be wearing two hearing devices. Indeed, among other advantages, administering a compound of formula (I), for example paliroden or xaliproden, or a pharmaceutically acceptable salt and/or solvate thereof, to a subject wearing at least one hearing device, leads to an improvement of speech-in-noise intelligibility.

According to another embodiment, the compound of formula (I) or a pharmaceutically acceptable salt and/or solvate thereof is administered to the subject during a cochlear surgery, for example during a cochlear implant surgery. Indeed, administering a compound of formula (I), for example paliroden or xaliproden, or a pharmaceutically acceptable salt and/or solvate thereof, to a subject during a cochlear surgery, for example during a cochlear implant surgery, allowing better preservation of residual hearing and/or facilitating rehabilitation post-surgery (better and/or faster post-surgery rehabilitation). Indeed, cochlear implant surgery is generally safe; however, risks of cochlear implantation include loss of residual hearing.

The inventors have surprisingly discovered that administering a compound of formula (I), for example paliroden or xaliproden, or a pharmaceutically acceptable salt and/or solvate thereof, leads to an improvement of the hearing function materialized by a decrease in pure-tone audiometric threshold and/or ABR threshold shift of at least 10 dB at three contiguous frequencies.

In addition, the inventors have surprisingly discovered that administering a compound of formula (I), for example paliroden or xaliproden, or a pharmaceutically acceptable salt and/or solvate thereof, leads to an improvement of speech intelligibility in silence materialized by a 10% improvement in word recognition score (number of correctly identified words in a list of 50 words presented at 30 dB above audiometric threshold).

In addition, the inventors have surprisingly discovered that administering a compound of formula (I), for example paliroden or xaliproden, or a pharmaceutically acceptable salt and/or solvate thereof, leads to an improvement of speech-in-noise intelligibility materialized by a reduction of 4 dB in the SNR (signal-to-noise ratio) necessary to achieve 50% correct word understanding.

Furthermore, the inventors have surprisingly discovered that administering a compound of formula (I), for example paliroden or xaliproden, or a pharmaceutically acceptable salt and/or solvate thereof, exhibits a beneficial effect following noise exposure with ABR threshold shifts reduction.

### Administration

The compound of formula (I), for example paliroden or xaliproden, or a pharmaceutically acceptable salt and/or solvate thereof, may be administered to the subject by any suitable administration route. Preferably, the compound of formula (I), for example paliroden or xaliproden, or a pharmaceutically acceptable salt and/or solvate thereof, is administered to the subject by any administration route suitable for allowing said compound to reach the inner ear. More preferably, the route suitable for allowing said compound to reach the inner ear is a route selected from the group consisting of a systemic route and a local route. The systemic route may be selected from the group consisting of oral route, intraperitoneal route, intravenous route, intramuscular route, intraarterial route and subcutaneous route. The local route may be selected from the group consisting of external ear route on the eardrum, trans-tympanic route, intrabullar route, intracochlear route and round window niche route.

Thus, the compound of formula (I), for example paliroden or xaliproden, or a pharmaceutically acceptable salt and/or solvate thereof, may be administered to the subject by an administration route selected from the group consisting of external ear route on the eardrum, trans-tympanic route, intrabullar route, intracochlear route, round window niche route, oral route, intraperitoneal route, intravenous route, intramuscular route, intraarterial route and subcutaneous route.

Advantageously, the compound of formula (I), for example paliroden or xaliproden, or a pharmaceutically acceptable salt and/or solvate thereof is administered to the subject by trans-tympanic route or by oral route. More advantageously, the administration by trans-tympanic route consists in an injection between the eardrum and the round window by means of a needle which crosses the eardrum.

In one embodiment, the compound of formula (I), for example paliroden or xaliproden, or a pharmaceutically acceptable salt and/or solvate thereof is administered to the subject by trans-tympanic route. In a preferred embodiment, the compound of formula (I) or a pharmaceutically acceptable salt and/or solvate thereof is administered to the subject by trans-tympanic route in one ear, for example the right ear or the left ear. In another preferred embodiment, the compound of formula (I) or a pharmaceutically acceptable salt and/or solvate thereof is administered to the subject by trans-tympanic route in both ears. Advantageously, the administration by trans-tympanic route consists in an injection between the eardrum and the round window by means of a needle which crosses the eardrum.

In another embodiment, the compound of formula (I), for example paliroden or xaliproden, or a pharmaceutically acceptable salt and/or solvate thereof is administered to the subject by oral route. The oral route has the advantages of promoting compliance by the subject and facilitating administration, especially when the subject is a non-human mammal, such as a cat or dog.

According to an embodiment, the compound of formula (I), for example paliroden or xaliproden, or a pharmaceutically acceptable salt and/or solvate thereof is administered to the subject by trans-tympanic route at a dose per ear ranging from 10 µg to 400 mg, preferably from 0.1 mg to 10 mg, more preferably from 1 mg to 10 mg, even more preferably from 4 mg to 6 mg, even more preferably about 5 mg. Advantageously, the administration by trans-tympanic route consists in an injection between the eardrum and the round window by means of a needle which crosses the eardrum.
According to an embodiment, the compound of formula (I) or a pharmaceutically acceptable salt and/or solvate thereof is administered to the subject by trans-tympanic route at a dose per ear ranging from 10 µg to 400 mg, preferably from 0.1 mg to 20 mg, more preferably from 1 mg to 20 mg, even more preferably from 8 mg to 12 mg, even more preferably about 10 mg.
According to an embodiment, the compound of formula (I) or a pharmaceutically acceptable salt and/or solvate thereof is administered to the subject by trans-tympanic route at a dose per ear ranging from 10 µg to 400 mg, preferably from 1 mg to 30 mg, more preferably from 5 mg to 25 mg, even more preferably from 17 mg to 23 mg, even more preferably about 20 mg.
According to an embodiment, the compound of formula (I) or a pharmaceutically acceptable salt and/or solvate thereof is administered to the subject by trans-tympanic route at a dose per ear ranging from 10 µg to 400 mg, preferably from 1 mg to 300 mg, more preferably from 50 mg to 200 mg, even more preferably from 100 mg to 150 mg, even more preferably about 125 mg.
According to an embodiment, the compound of formula (I) or a pharmaceutically acceptable salt and/or solvate thereof is administered to the subject by trans-tympanic route at a dose per ear of about 10 µg, 0.1 mg, 0.5 mg, 0.75 mg, 1 mg, 1.25 mg, 1.5 mg, 1.75 mg, 2 mg, 2.25 mg, 2.5 mg, 2.75 mg, 3 mg, 3.25 mg, 3.5 mg, 3.75 mg, 4 mg, 4.25 mg, 4.5 mg, 4.75 mg, 5 mg, 5.25 mg, 5.5 mg, 5.75 mg, 6 mg, 6.25 mg, 6.5 mg, 6.75 mg, 7 mg, 7.25 mg, 7.5 mg, 7.75 mg, 8 mg, 8.25 mg, 8.5 mg, 8.75 mg, 9 mg, 9.25 mg, 9.5 mg, 9.75 mg, 10 mg, 10.25 mg, 10.5 mg, 10.75 mg, 11 mg, 11.25 mg, 11.5 mg, 12 mg, 12.5 mg, 13 mg, 13.5 mg,14 mg, 14.5 mg, 15 mg, 15.5 mg, 16 mg, 16.5 mg, 17 mg, 17.5 mg, 18 mg, 18.5 mg, 19 mg, 19.5 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 260 mg, 270 mg, 280 mg, 290 mg, 300 mg, 310 mg, 320 mg, 330 mg, 340 mg, 350 mg, 360 mg, 370 mg, 380 mg, 390 mg or 400 mg.
Said dose may be administered in one ear, for example the right ear or the left ear, between once a month and once every 12 months. Said dose may also be administered in both ears between once a month and once every 12 months.
Advantageously, the administration by trans-tympanic route consists in an injection between the eardrum and the round window by means of a needle which crosses the eardrum.

According to an embodiment, the compound of formula (I), for example paliroden or xaliproden, or a pharmaceutically acceptable salt and/or solvate thereof is administered to the subject by oral route at a dose ranging from 100 µg to 4 mg, preferably from 1 mg to 4 mg, more preferably from 2 mg to 4 mg, even more preferably about 3 mg, said dose being administered between twice a day and once a week, advantageously said dose being administered once a day. This oral dose may in particular be administered to a human.

According to an embodiment, the compound of formula (I) or a pharmaceutically acceptable salt and/or solvate thereof is administered by oral route at a dose ranging from 1 mg/kg/day to 10 mg/kg/day, preferably from 3 mg/kg/day to 7 mg/kg/day, more preferably about 5 mg/kg/day, said dose being administered between twice a day and once a week, advantageously said dose being administered once a day. This oral dose may in particular be administered to a non-human subject.

According to an embodiment, the compound of formula (I), for example paliroden or xaliproden, or a pharmaceutically acceptable salt and/or solvate thereof is administered in a micronized form.

According to an embodiment, the compound of formula (I), for example paliroden or xaliproden, or a pharmaceutically acceptable salt and/or solvate thereof is administered in an atomized form.

In one embodiment, the subject is a mammalian animal with an eardrum. In a preferred embodiment, said mammalian animal with an eardrum is a human. In another preferred embodiment, said mammalian animal with an eardrum is non-human, preferably a non-human mammalian animal with an eardrum selected from the group consisting of a cat, a dog, a horse and a non-human primate such as a monkey.

In a more preferred embodiment, the subject is a dog.

In a more preferred embodiment, the subject is a human, advantageously a human over 40 years.

In a more preferred embodiment, the subject is diabetic (type 1 diabetes or type 2 diabetes, preferably type 2 diabetes).

### Pharmaceutical composition

The present invention also relates to a pharmaceutical composition comprising a compound of formula (I) or a pharmaceutically acceptable salt and/or solvate thereof as defined above and at least one pharmaceutically acceptable excipient selected from the group consisting of poloxamers, polyethoxylated castor oil, phospholipids, triglycerides and any combination thereof.

The present invention also relates to a pharmaceutical composition comprising a compound of formula (I) or a pharmaceutically acceptable salt and/or solvate thereof as defined above, wherein said pharmaceutical composition is a thermoreversible gel, and preferably, said pharmaceutical composition is a pharmaceutical trans-tympanic composition.

Advantageously, said pharmaceutical composition is a pharmaceutical trans-tympanic composition, i.e. a pharmaceutical composition intended to be administered by trans-tympanic route.

In one embodiment, said pharmaceutical composition comprises from 0.1 to 15 mg of the compound of formula (I) or a pharmaceutically acceptable salt and/or solvate thereof per mL of said pharmaceutical composition.

In one embodiment, said pharmaceutical composition comprises from 0.1 to 15 mg of paliroden or a pharmaceutically acceptable salt and/or solvate thereof per mL of said pharmaceutical composition.

In one embodiment, said pharmaceutical composition comprises from 0.1 to 15 mg of xaliproden or a pharmaceutically acceptable salt and/or solvate thereof per mL of said pharmaceutical composition.

According to one embodiment, the at least one pharmaceutically acceptable excipient is selected from the group consisting of poloxamers, polyethoxylated castor oil, triglycerides, combinations of phospholipids and triglycerides, and any combination thereof.

According to one embodiment, the at least one pharmaceutically acceptable excipient is selected from the group consisting of poloxamers, triglycerides, and any combination thereof. According to one embodiment, the at least one pharmaceutically acceptable excipient is selected from the group consisting of poloxamers, triglycerides, combinations of phospholipids and triglycerides, and any combination thereof. In one embodiment, the triglycerides are polyoxyethylated.

According to one embodiment, the at least one pharmaceutically acceptable excipient is at least one poloxamer, preferably said poloxamer being selected from the group consisting of poloxamer 407, poloxamer 188 and any combination thereof.

According to a preferred embodiment, the at least one pharmaceutically acceptable excipient is a mixture of polyoxyethylated triglycerides, obtained by reacting castor oil with ethylene oxide in a molar ratio of 1:35. For example, the at least one pharmaceutically acceptable excipient may be the commercialized product Cremophor EL^{®}, which CAS number is 61791-12-6.

According to a preferred embodiment, the at least one pharmaceutically acceptable excipient is a combination of poloxamers and a mixture of polyoxyethylated triglycerides, preferably a mixture of polyoxyethylated triglycerides obtained by reacting castor oil with ethylene oxide in a molar ratio of 1:35 such as Cremophor EL^{®}. More preferably, the pharmaceutical composition comprising said excipients is a thermoreversible gel.

According to a preferred embodiment, the at least one pharmaceutically acceptable excipient is a combination of poloxamer 407 and a mixture of polyoxyethylated triglycerides, preferably a mixture of polyoxyethylated triglycerides obtained by reacting castor oil with ethylene oxide in a molar ratio of 1:35 such as Cremophor EL^{®}.

According to another preferred embodiment, the at least one pharmaceutically acceptable excipient is a combination of poloxamer 407, poloxamer 188 and a mixture of polyoxyethylated triglycerides, preferably a mixture of polyoxyethylated triglycerides obtained by reacting castor oil with ethylene oxide in a molar ratio of 1:35 such as Cremophor EL^{®}.

According to another preferred embodiment, the at least one pharmaceutically acceptable excipient comprises or consists of a combination of:
- from 7% w/w to 15% w/w of poloxamer 407,
- from 7% w/w to 12% w/w of poloxamer 188, and
- from 17% w/w to 20% w/w, preferably about 20% w/w, of a mixture of polyoxyethylated triglycerides, preferably a mixture of polyoxyethylated triglycerides obtained by reacting castor oil with ethylene oxide in a molar ratio of 1:35 such as Cremophor EL^{®},
wherein the percentages are expressed in relation to the total volume of the pharmaceutical composition.

According to one embodiment, the at least one pharmaceutically acceptable excipient is a combination of phospholipids and triglycerides. Preferably, said combination of phospholipids and triglycerides comprises phosphatidylcholine and medium-chain triglycerides such as caprylic/capric triglycerides. For example, said combination of phospholipids and triglycerides may be the commercialized product Phosal@ 53 MCT.

According to an embodiment, the compound of formula (I) or a pharmaceutically acceptable salt and/or solvate thereof is in a micronized form in the pharmaceutical composition.

According to an embodiment, the compound of formula (I), for example paliroden or xaliproden, or a pharmaceutically acceptable salt and/or solvate thereof is in an atomized form in the pharmaceutical composition.

According to an embodiment, said pharmaceutical composition is a sustained release composition.

In one embodiment, said pharmaceutical composition has a viscosity from 500 to 5000 mPa.s (or cP). In a preferred embodiment, said pharmaceutical composition has a viscosity from 600 to 800 mPa.s, preferably from 650 to 800 mPa.s, preferably from 650 to 750 mPa.s, more preferably about 700 mPa.s. In another preferred embodiment, said pharmaceutical composition has a viscosity from 4000 to 5000 mPa.s, preferably from 4500 to 5000 mPa.s, more preferably about 4750 mPa.s. The viscosity is the dynamic viscosity, measured with a viscometer using the capillary tube method, at 25°C.

Such a pharmaceutical composition allows a compound of formula (I) or a pharmaceutically acceptable salt and/or solvate thereof, such as paliroden or xaliproden, to be administered by trans-tympanic administration, in particular by injection between the eardrum and the round window by means of a needle which crosses the eardrum. This trans-tympanic administration, in particular by injection between the eardrum and the round window by means of a needle which crosses the eardrum, has the advantage of allowing a higher concentration of the compound of formula (I), such as paliroden or xaliproden, or a pharmaceutically acceptable salt and/or solvate thereof, in the inner ear fluid to be reached (above 500 ng/mL, i.e. approximately 1µM), than the oral administration, while reducing the systemic exposure compared to the oral administration.

The administration of a mucoadhesive composition, by trans-tympanic route, in particular by injection between the eardrum and the round window by means of a needle which crosses the eardrum, allows said composition to adhere to a mucous membrane of the ear, in particular to the mucous membrane of the middle ear. Then, the active agent diffuses through the round window to the cochlea. Thus, the muco-adhesive feature of the composition ensures that the active agent reaches the cochlea. Excipients such as polyethoxylated castor oil, triglycerides and combinations of triglyderides and phospholipids allow to confer the mucoadhesive feature to a composition.

Without wishing to be bound by any theory, it seems that the viscosity of the pharmaceutical composition according to the invention is involved in allowing this composition to remain longer in the middle ear after injection between the eardrum and the round window by means of a needle which crosses the eardrum. Therefore, it allows the compound of formula (I), such as paliroden or xaliproden, or a pharmaceutically acceptable salt and/or solvate thereof, to diffuse for a longer through the round window to the cochlea, ensuring a greater amount of the compound of formula (I), such as paliroden or xaliproden, or a pharmaceutically acceptable salt and/or solvate thereof, to reach the cochlea.

### Pharmaceutical composition for use

The present invention also relates to a pharmaceutical composition for use in the treatment of a hearing disease in a subject, said pharmaceutical composition comprising a compound of formula (I) or a pharmaceutically acceptable salt and/or solvate thereof as defined above and at least one pharmaceutically acceptable excipient.

The present invention also relates to a method of treating a hearing disease by administering to a subject in need thereof an effective amount of a pharmaceutical composition, said pharmaceutical composition comprising a compound of formula (I) or a pharmaceutically acceptable salt and/or solvate thereof as defined above and at least one pharmaceutically acceptable excipient. The present invention also relates to the use of a pharmaceutical composition for the manufacture of a medicament for the treatment of a hearing disease in a subject, said pharmaceutical composition comprising a compound of formula (I) or a pharmaceutically acceptable salt and/or solvate thereof as defined above.

The present invention also relates to the use of a pharmaceutical composition for the treatment of a hearing disease in a subject, said pharmaceutical composition comprising a compound of formula (I) or a pharmaceutically acceptable salt and/or solvate thereof as defined above.

Advantageously, said hearing disease is as defined above in the present description.

Said pharmaceutical composition may be administered to the subject by any suitable administration route. Preferably, said pharmaceutical composition is administered to the subject by any administration route suitable for allowing the compound of formula (I) or a pharmaceutically acceptable salt and/or solvate thereof to reach the inner ear. More preferably, the route suitable for allowing the compound of formula (I) or a pharmaceutically acceptable salt and/or solvate thereof to reach the inner ear is a route selected from the group consisting of a systemic route and a local route. The systemic route may be selected from the group consisting of oral route, intraperitoneal route, intravenous route, intramuscular route, intraarterial route and subcutaneous route. The local route may be selected from the group consisting of external ear route on the eardrum, trans-tympanic route, intrabullar route, intracochlear route and round window niche route.

Thus, said pharmaceutical composition may be administered to the subject by an administration route selected from the group consisting of external ear route on the eardrum, trans-tympanic route, intrabullar route, intracochlear route, round window niche route, oral route, intraperitoneal route, intravenous route, intramuscular route, intraarterial route and subcutaneous route.

Advantageously, said pharmaceutical composition is administered to the subject by trans-tympanic route or by oral route. More advantageously, the administration by trans-tympanic route consists in an injection between the eardrum and the round window by means of a needle which crosses the eardrum.

According to a first embodiment, said pharmaceutical composition is administered to the subject by trans-tympanic route. Preferably, said pharmaceutical composition is administered to the subject by trans-tympanic route in one ear, for example the right ear or the left ear. Alternatively, said pharmaceutical composition is administered to the subject by trans-tympanic route in both ears. Advantageously, the administration by trans-tympanic route consists in an injection between the eardrum and the round window by means of a needle which crosses the eardrum. Preferably, the pharmaceutical composition that is administered to the subject by trans-tympanic route, is as defined above in part "pharmaceutical composition" of the present description.

According to a second embodiment, said pharmaceutical composition is administered to the subject by oral route. Preferably, the pharmaceutical composition that is administered to the subject by oral route is in a galenic form selected from the group consisting of drinkable suspensions, granules, capsules and tablets. Preferably, the at least one pharmaceutically acceptable excipient of the pharmaceutical composition that is administered to the subject by oral route is selected from the group consisting of lactose monohydrate, microcrystalline cellulose, corn starch, citric acid, hypromellose, croscarmellose sodium, magnesium stearate, methylcellulose and arabic gum. More preferably, the at least one pharmaceutically acceptable excipient of the pharmaceutical composition that is administered to the subject by oral route comprises a combination of:
- lactose monohydrate, microcrystalline cellulose, corn starch as diluents,
- citric acid as antioxidant,
- hypromellose as binding agent,
- croscarmellose sodium as disintegrant,
- magnesium stearate as lubricant, and
- methylcellulose or arabic gum as suspending agents.

The oral galenic form may be coated, for example with an aqueous film coating system comprising polyvinyl alcohol, such as Opadry^{®} II (for example Opadry^{®} II 32K28708 white).

The administration to a subject by oral route of a pharmaceutical composition comprising a compound of formula (I), such as paliroden or xaliproden, or pharmaceutically acceptable salt and/or solvate thereof allows said compound of formula (I) or a pharmaceutically acceptable salt and/or solvate thereof, such as paliroden or xaliproden, to reach concentrations in the inner ear fluid that are higher than 5 ng/mL (i.e. about 10 nM).

All the features described above for the compound for use (part "compound for use") and all the features described above for the pharmaceutical composition (part "pharmaceutical composition") apply *mutatis mutandis* for the pharmaceutical composition for use. In particular, all the features described above regarding the compound of formula (I) or a pharmaceutically acceptable salt and/or solvate thereof, the indications, the administration (notably the administration routes, the doses, the form of the compound of formula (I) or a pharmaceutically acceptable salt and/or solvate thereof, the subject), the concentration of the compound of formula (I) or a pharmaceutically acceptable salt and/or solvate thereof in the pharmaceutical composition, the excipients, the form of the compound of formula (I) or a pharmaceutically acceptable salt and/or solvate thereof in the pharmaceutical composition, the sustained release feature and the viscosity of the pharmaceutical composition, apply *mutatis mutandis* for the pharmaceutical composition for use.

### BRIEF DESCRIPTION OF THE DRAWINGS

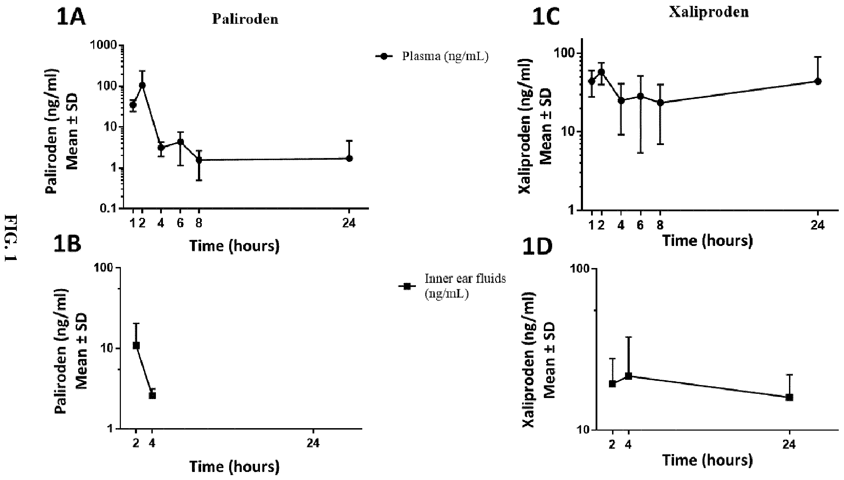

**Figure 1** is a graph showing the pharmacokinetic profiles of paliroden in plasma (1A) and IEF (1B) and xaliproden in plasma (1C) and IEF (1D) following administration at 30 mg/kg of paliroden and 20 mg/kg xaliproden to male Wistar rats by single oral gavage. Concentration mean values and standard deviations (in ng/ml) are reported in log scale across time for each compound and matrix.

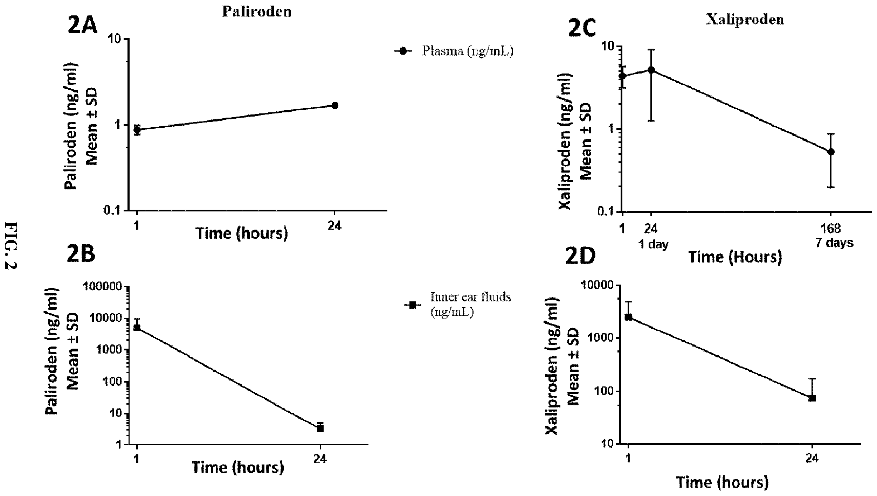

**Figure 2** is a graph showing the pharmacokinetic profiles of paliroden in plasma (2A) and IEF (2B) and xaliproden in plasma (2C) and IEF (2D) following a single trans-tympanic administration, by injection between the eardrum and the round window by means of a needle which crosses the eardrum, of 243 µg of paliroden and 192 µg of xaliproden to the middle ear of male Wistar rats. Concentration mean values and standard deviations (in ng/mL) are reported in log scale across time for each compound and matrix.

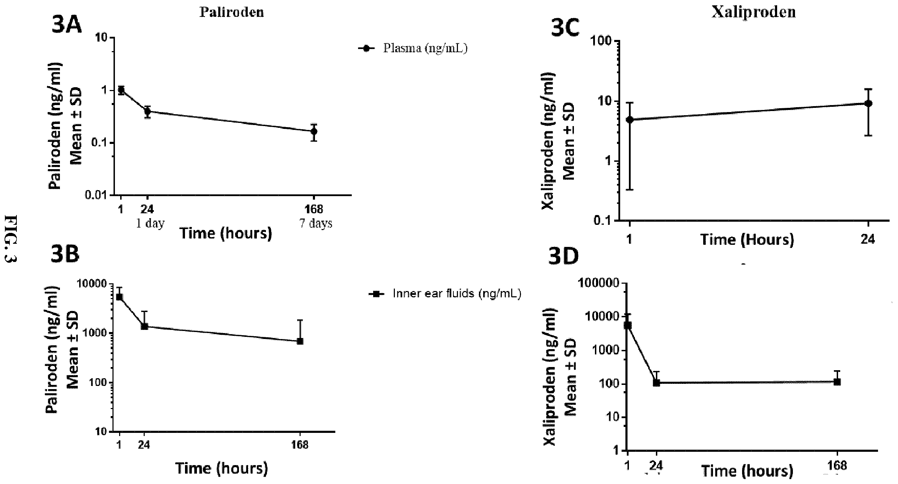

**Figure 3** is a graph showing the pharmacokinetic profiles of paliroden in plasma (3A) and IEF (3B) and xaliproden in plasma (3C) and IEF (3D) following a single trans-tympanic administration, by injection between the eardrum and the round window by means of a needle which crosses the eardrum, of 300 µg of paliroden and 150 µg of xaliproden to the middle ear of male Wistar rats. Concentration mean values and standard deviations (in ng/mL) are reported in log scale across time for each compound and matrix.

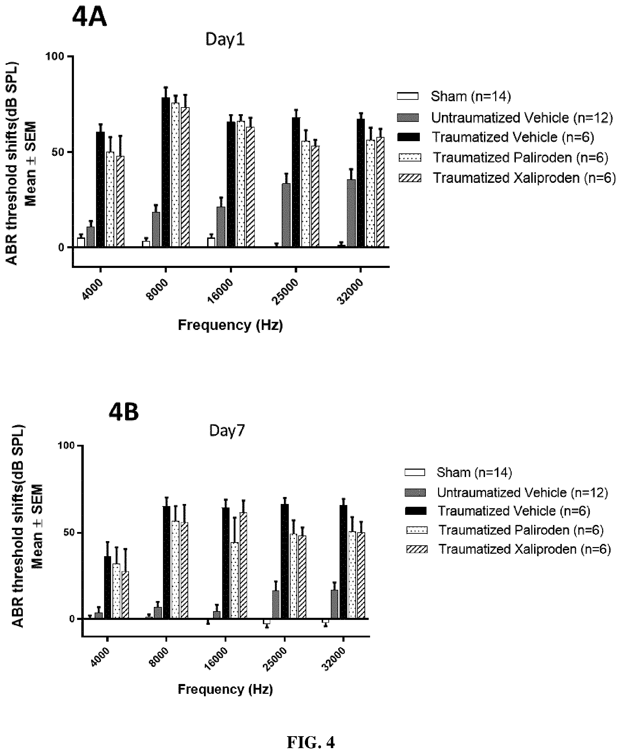

**Figure 4** is a histogram showing auditory thresholds shifts (in dB) of control rats (sham group, untraumatized vehicle group and traumatized vehicle group) and rats treated with paliroden or xaliproden, at 1 day (4A) or 7 days (4B) post noise exposure and treatment, in comparison to baseline (prior to noise exposition).

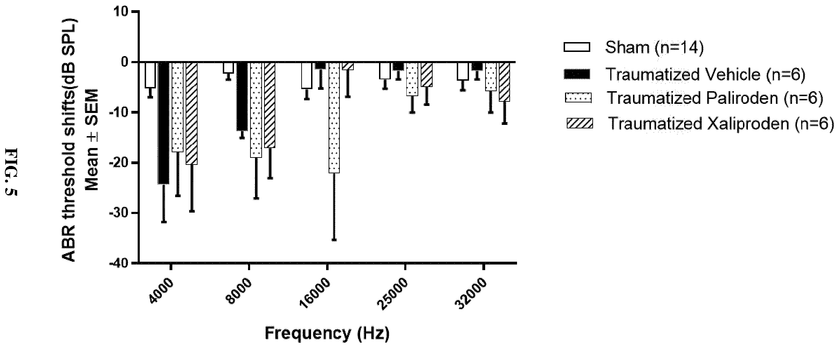

**Figure 5** is a histogram showing the comparison of hearing recovery (in dB) achieved between 1 day and 7 days following noise-trauma and trans-tympanic treatment, in control rats (sham group and traumatized vehicle group) and rats treated in both ears with paliroden or xaliproden.

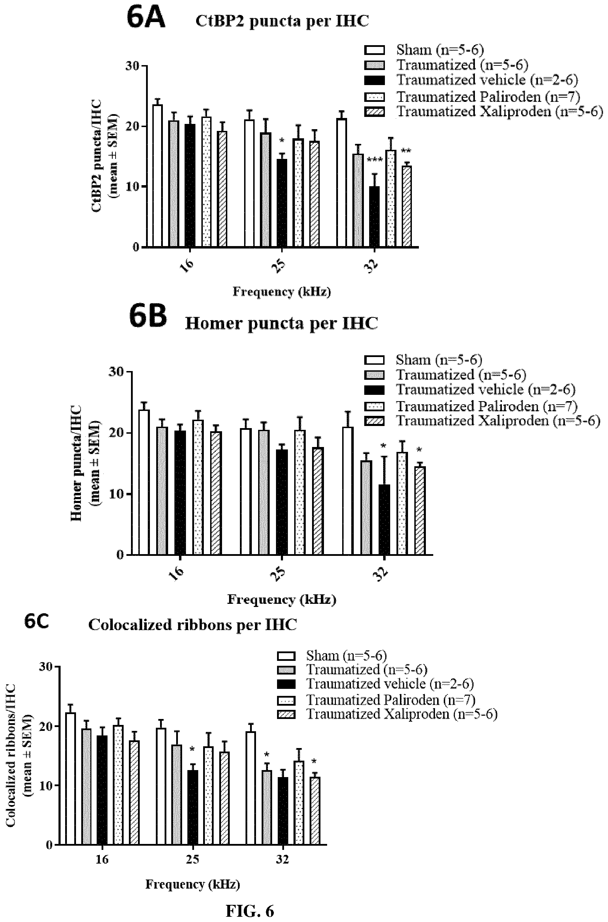

**Figure 6** is a histogram showing the number of CtBP2 spots (pre-synaptic elements) per inner hair cell (6A), Homer (post-synaptic elements) per inner hair cell (6B) and colocalized ribbons per inner hair cells (6C), detected and counted following immunolabelling of dissected cochlea sampled at T+28Day following noise exposure in control rats (sham, traumatized and traumatized vehicle group) in comparison to rats treated in both ears with paliroden or xaliproden immediately after noise exposure.

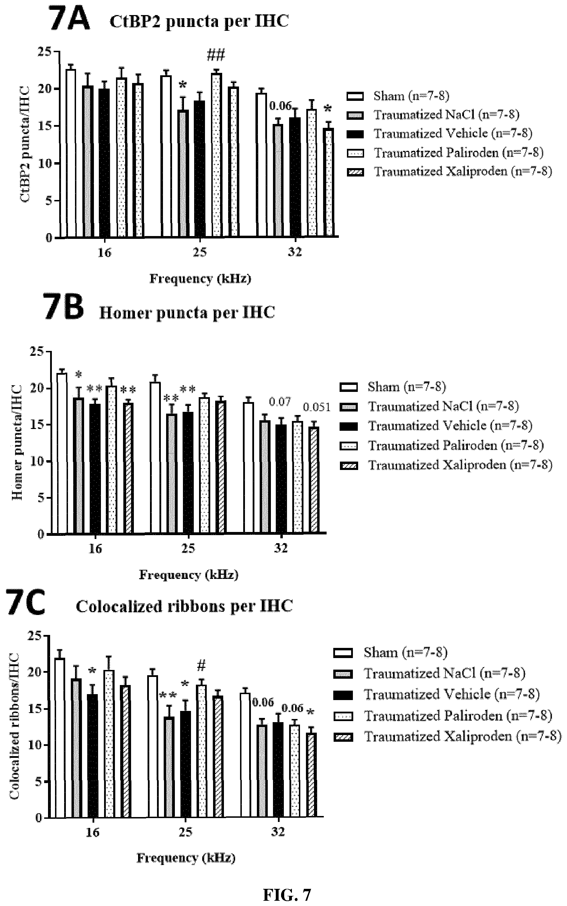

**Figure 7** is a histogram showing the number of CtBP2 spots (pre-synaptic elements) per inner hair cell (7A), Homer (post-synaptic elements) per inner hair cell (6B) and colocalized ribbons per inner hair cells (6C), detected and counted following immunolabelling of dissected cochlea sampled at T+28Day following noise exposure in control rats (sham, traumatized NaCl and traumatized vehicle group) in comparison to rats treated in both ears with paliroden or xaliproden 2 days after noise exposure.

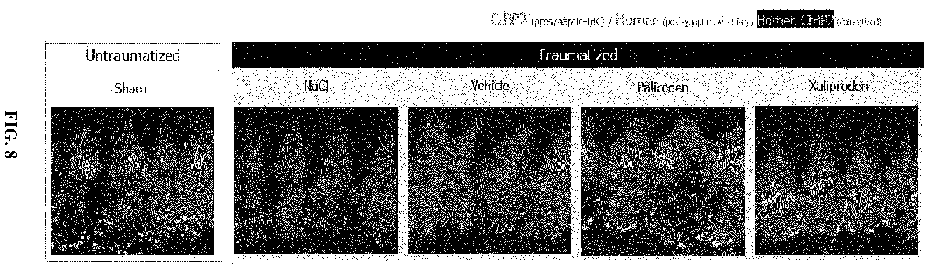

**Figure 8** is a representative panel showing a single image acquired in the cochlea region coding 25kHz for one animal of each group (Sham, Traumatized NaCl, Traumatized Vehicle, Traumatiezd Paliroden and Traumatized Xaliproden). Differences are shown on the number of CtBP2 (pre-synaptic elements) labelled in green, Homer (post-synaptic elements) labelled in red and co-localized ribbon merged in yellow, in the different treatment groups.

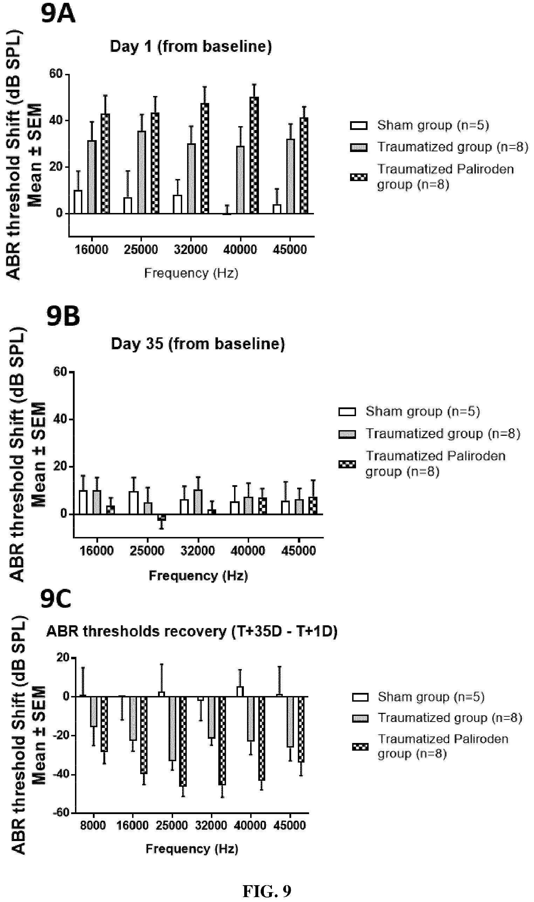

**Figure 9** is a histogram showing auditory thresholds shifts (in dB) of control mice (sham and traumatized groups) and mice treated with paliroden, at 1 day (9A) or 35 days (9B) post noise exposure, with treatment occurring just after the day 1 measure, in comparison to baseline (prior to noise exposition). Hearing threshold recovery (in dB) achieved post-treatment between Day 1 and Day35 are compared in control mice (sham and traumatized group) and mice treated in both ears with paliroden (9C).

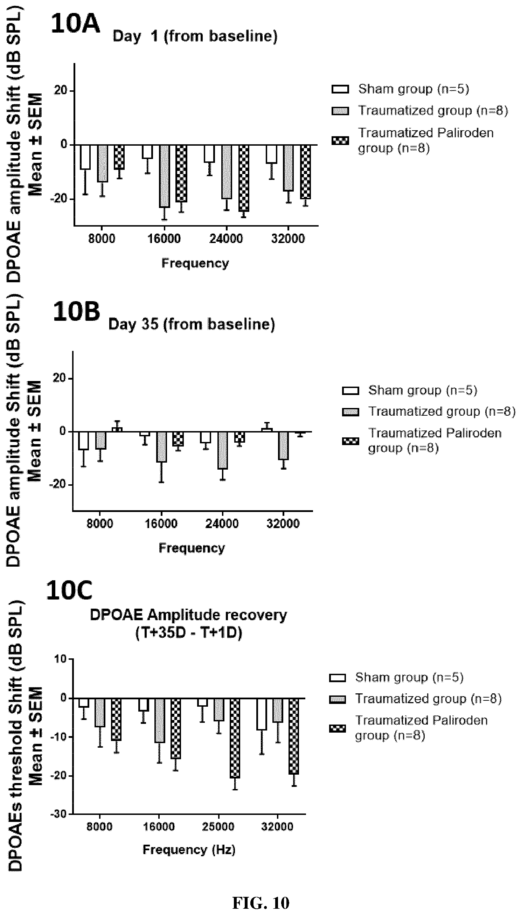

**Figure 10** is a histogram showing DPOAE amplitude shifts (in dB) of control mice (sham and traumatized groups) and mice treated with paliroden, at 1 day (10A) or 35 days (10B) post noise exposure, with treatment occurring just after the day 1 measure, in comparison to baseline (prior to noise exposition). Amplitude recovery (in dB) achieved post-treatment between Day 1 and Day35 are compared in control mice (sham and traumatized group) and mice treated in both ears with paliroden (10C).

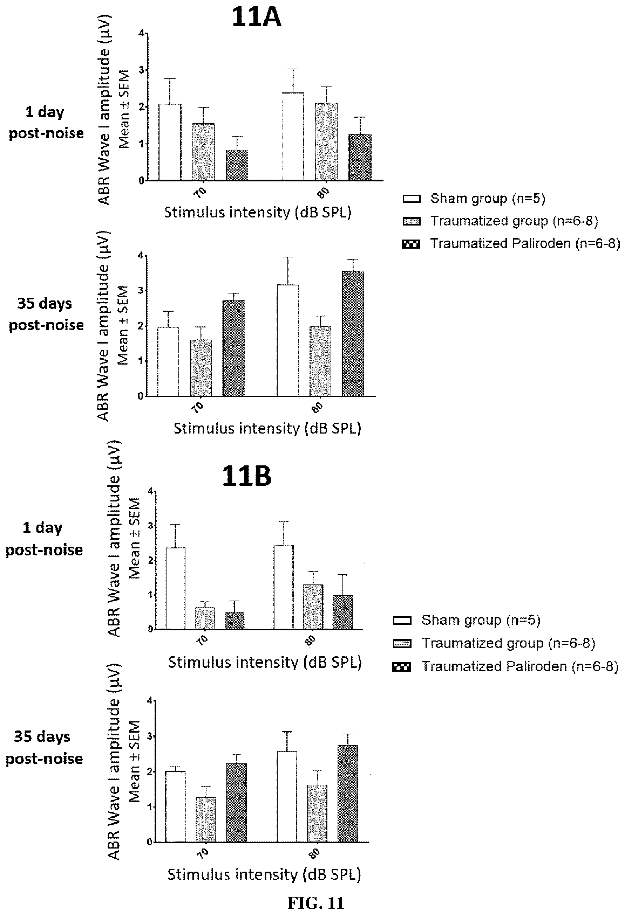

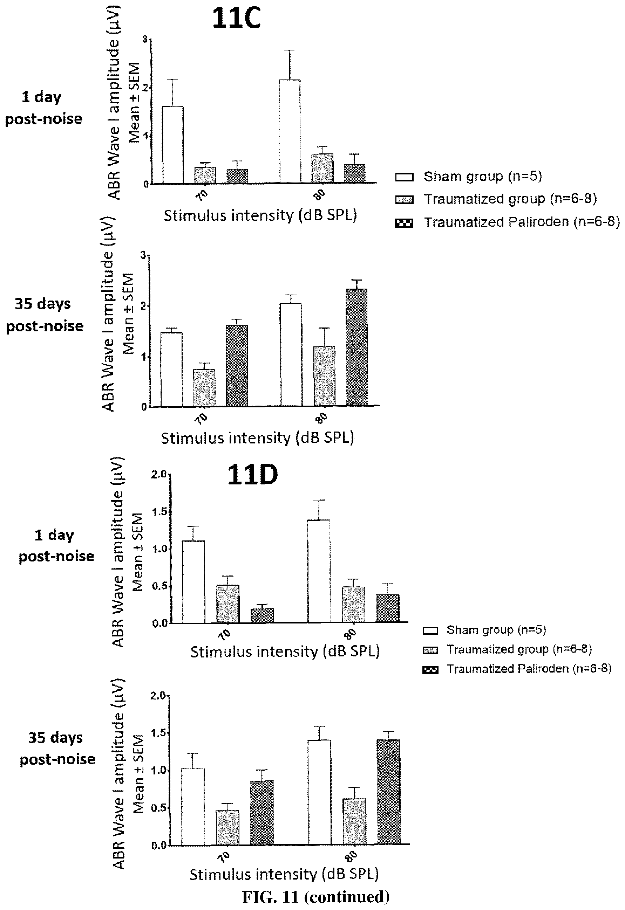

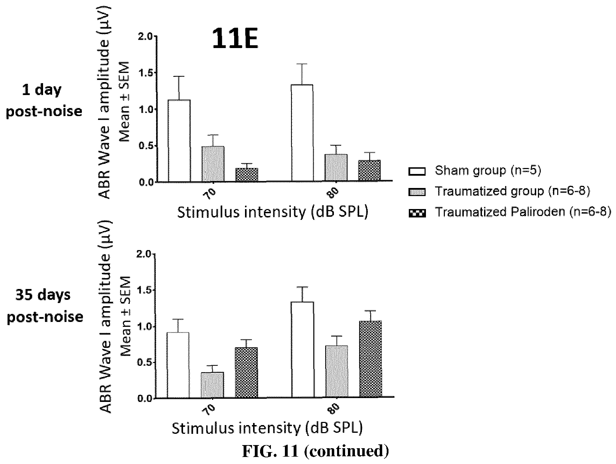

**Figure 11** is a histogram comparing ABR Wave I amplitude (in µV), i.e. the activity of the auditory nerve fibers, in control mice (sham and traumatized groups) and mice treated in both ears with paliroden, at day 1 (top row) versus day 35 (bottom row) post noise exposure, at all tested frequencies 16kHz (11A), 25kHz (11B), 32kHz (11C), 40kHz (11D) and 45kHz (11E).

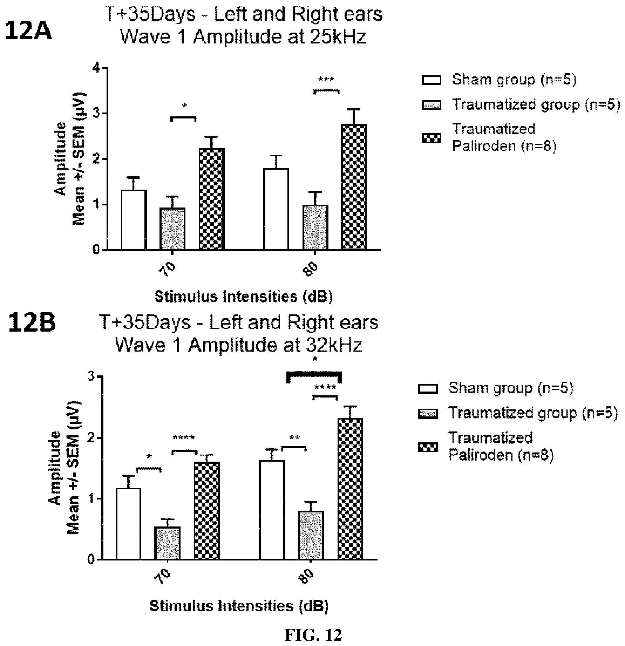

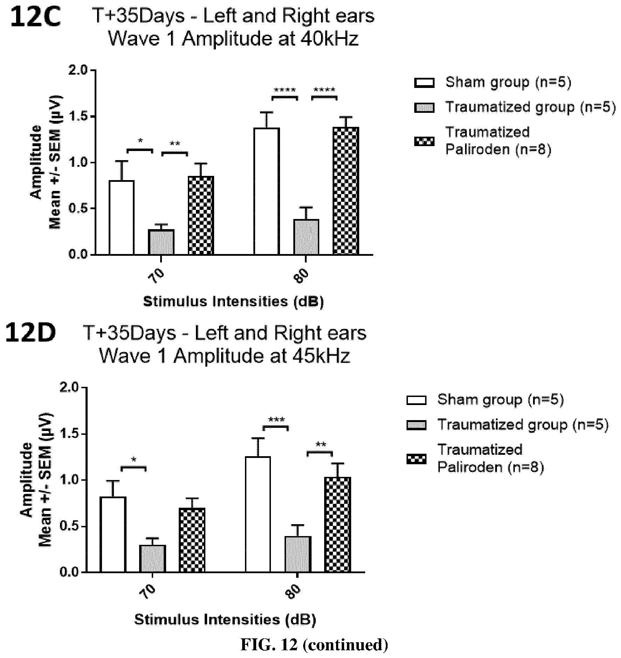

**Figure 12** is a histogram comparing ABR Wave I amplitude (in µV), i.e. the activity of the auditory nerve fibers, in control mice (sham and traumatized groups) and mice treated in both ears with paliroden, at day 35 post noise exposure, at high frequencies 25kHz (12A), 32kHz (12B), 40kHz (12C) and 45kHz (12D) in a subset of responding animals.

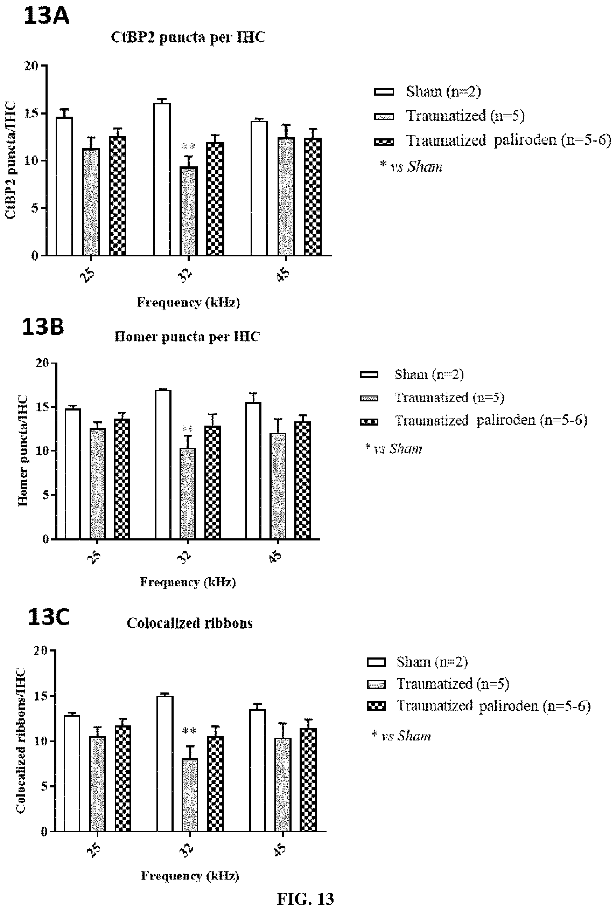

**Figure 13** is a histogram showing the number of CtBP2 spots (pre-synaptic elements) per inner hair cell (13A), Homer (post-synaptic elements) per inner hair cell (13B) and colocalized ribbons per inner hair cells (13C), detected and counted following immunolabelling of dissected cochlea sampled at T+35 day following noise exposure in control mice (sham and traumatized group) in comparison to mice treated in both ears with paliroden.

### EXAMPLES

The present invention is further illustrated by the following examples.

### Example 1: Inner ear exposition of paliroden and xaliproden following oral administration to male Wistar rats

### Materials and Methods

Two compositions were prepared: composition A and composition B. Composition A is an aqueous solution comprising:
- 6 mg/mL (free base equivalent) of fumarate salt of 1-[2-(4-biphenylyl)ethyl]-4-(3-trifluoromethylphenyl)-1,2,3,6-tetrahydropyridine (=paliroden fumarate), and
- 0.6% w/v of methylcellulose with respect to the total volume of the aqueous solution.
Composition B is an aqueous solution comprising:
- 4 mg/mL (free base equivalent) of hydrochloride salt of 1-[2-(2-naphthyl)ethyl]-4-(3-trifluoromethylphenyl)-1,2,3,6-tetrahydropyridine (=xaliproden hydrochloride), and
- 10% w/v of arabic gum with respect to the total mass of the aqueous solution.

Composition A and composition B were administered to male Wistar rats (5 animals per active agent and timepoint), as described below.

Composition A was administered to 15 male Wistar rats, once, at time T0, via oral injection by gavage, at a dose of 5 mL of composition A per kilogram of the animal, i.e. a dose of 30 mg of paliroden fumarate per kilogram of the animal. The concentration of paliroden in rats plasma and in rats inner ear fluid (IEF) was measured at 6 timepoints, 5 rats being used at each timepoint for the concentration measurements (in the plasma and optionally in the IEF). The timepoints are T0+1hour, T0+2hours, T0+4hours, T0+6hours, T0+8hours and T0+24hours for the concentration measurement in the rats plasma. The timepoints are T0+2hours, T0+4hours and T0+24hours for the concentration measurement in the rats inner ear fluid, as part of a terminal procedure including cochlea sampling and inner ear fluids collection from both ears of the rats. Bioanalytical methods were validated in both matrices (lithium heparin plasma and inner ear fluid) with a range of 0.25 to 500 ng/mL in plasma and 0.25 to 100 ng/mL in inner ear fluids.

Composition B was administered to 15 others male Wistar rats, once, at time T0, via oral injection by gavage, at a dose of 5 mL of composition B per kilogram of the animal, i.e. a dose of 20 mg of xaliproden hydrochloride per kilogram of the animal. The concentration of xaliproden in rats plasma and in rats inner ear fluid (IEF) was measured at 6 timepoints, 5 rats being used at each timepoint for the concentrations measurements (in the plasma and optionally in the IEF). The timepoints are T0+1hour, T0+2hours, T0+4hours, T0+6hours, T0+8hours and T0+24hours for the concentration measurement in the rats plasma. The timepoints are T0+2hours, T0+4hours and T0+24hours for the concentration measurement in the rats inner ear fluid, as part of a terminal procedure including cochlea sampling and inner ear fluids collection from both ears of the rats. Bioanalytical methods were validated in both matrices (lithium heparin plasma and inner ear fluid) with a range of 0.25 to 500 ng/mL in plasma and 0.25 to 500 ng/mL in inner ear fluids.

### Results

The results are presented in **figure 1** and in the following **tables 1 and 2:**

**Table 1 - Bioanalytical results for paliroden (fumarate salt) in plasma and inner ear fluids following single oral administration to male Wistar rats at 30 mg/kg at T0**

| Timepoints | Concentration of paliroden (fumarate salt) (ng/mL) | | | | | |
|---|---|---|---|---|---|---|
| | Plasma | | | IEF | | |
| | Mean | SD | RSD (%) | Mean | SD | RSD (%) |
| T0 + 1 hour | 35.0 | 11.0 | 31.4 | - | - | - |
| T0 + 2 hours | 105.5 | 131.3 | 124.4 | 10.9 | 9.6 | 88.3 |
| T0 + 4 hours | 3.1 | 1.2 | 39.0 | 2.6 | 0.5 | 21.1 |
| T0 + 6 hours | 4.4 | 3.2 | 73.5 | - | - | - |
| T0 + 8 hours | 1.6 | 1.1 | 68.3 | - | - | - |
| T0 + 24 hours | 1.7 | 2.9 | 171.4 | BLQ | NA | NA |

| | | | | | | |
|---|---|---|---|---|---|---|
| SD = standard deviation. RSD = relative standard deviation. IEF = inner ear fluid. NA = not available. BLQ = below limit of quantification. | | | | | | |

**Table 2 - Bioanalytical results for xaliproden (hydrochloride salt) in plasma and inner ear fluids following single oral administration to male Wistar rats at 20 mg/kg at T0**

| Timepoints | Concentration of xaliproden (hydrochloride salt) (ng/mL) | | | | | |
|---|---|---|---|---|---|---|
| | Plasma | | | IEF | | |
| | Mean | SD | RSD (%) | Mean | SD | RSD (%) |
| T0 + 1 hour | 44.1 | 16.2 | 36.7 | - | - | - |
| T0 + 2 hours | 58.0 | 18.2 | 31.3 | 19.4 | 8.4 | 43.5 |
| T0 + 4 hours | 25.1 | 15.9 | 63.4 | 21.7 | 15.9 | 73.2 |
| T0 + 6 hours | 28.4 | 23.0 | 80.9 | - | - | - |
| T0 + 8 hours | 23.4 | 16.4 | 70.2 | - | - | - |
| T0 + 24 hours | 44.1 | 46.2 | 104.7 | 16.0 | 6.1 | 38.2 |

| | | | | | | |
|---|---|---|---|---|---|---|
| SD = standard deviation. RSD = relative standard deviation. IEF = inner ear fluid. | | | | | | |

As shown in **tables 1 and 2** and in **figure 1****,** paliroden and xaliproden salts, when administered by oral route, are able to cross the blood-labyrinth barrier and reach the inner ear. They are present in the IEF at concentrations ranging from about 2 ng/mL to about 20 ng/mL of IEF (approximately 4 to 40 nM).

### Example 2: Compositions of paliroden and xaliproden for trans-tympanic administration (by injection between the eardrum and the round window by means of a needle which crosses the eardrum)

### Materials and Methods

Eight compositions compatible with trans-tympanic injections were prepared: four thermoreversible gel compositions and four lipid-based mucoadhesive solutions. These eight compositions were prepared both with paliroden fumarate as active agent, and with xaliproden hydrochloride as active agent.

The four thermoreversible gel compositions comprising paliroden are as follows:

**Table 3**

| | Composition IP | Composition 2P | Composition 3P | Composition 4P |
|---|---|---|---|---|
| Poloxamer 407 | 15 % w/w | 17 % w/w | 11.2 % w/w | 7.2 % w/w |
| Poloxamer 188 | - | 10 % v/v | - | 8 % w/w |
| Cremophor EL^{®} | - | - | 20 % w/w | 20 % w/w |
| Buffering agent | Bicarbonate 0.1M | Phosphate 8mM pH=7.4 | Phosphate 8mM pH=7.4 | Phosphate 8mM pH=7.4 |
| pH | 8.3 | 4.8 | 4.9 | 4.7 |
| Micronized paliroden (fumarate salt) | 0.19 mg/mL | 0.59 mg/mL | 2.3 mg/mL | 10 mg/mL (= 1 % w/v) |

| | | | | |
|---|---|---|---|---|
| The percentages "% w/w" are expressed in relation to the total weight of each composition. Compositions 1P, 2P and 3P are solutions. Composition 4P is a suspension and the percentages "% w/v" are expressed in relation to the total volume of the composition. | | | | |

The four thermoreversible gel compositions comprising xaliproden are as follows:

**Table 4**

| | Composition 1X | Composition 2X | Composition 3X | Composition 4X |
|---|---|---|---|---|
| Poloxamer 407 | 15 % w/w | 17 % w/w | 11.2 % w/w | 7.2 % w/w |
| Poloxamer 188 | - | 10 % w/w | - | 8 % w/w |
| Cremophor EL^{®} | - | - | 20 % w/w | 20 % w/w |
| Buffering agent | Bicarbonate 0.1M | Phosphate 8mM | Phosphate 8mM | Phosphate 8mM |
| pH | 8.5 | 6 | 5.3 | 4.7 |
| Micronized xaliproden (hydrochloride salt) | 0.07 mg/ml | 0.12 mg/ml | 0.6 mg/ml | 5 mg/mL (= 0.5 % w/v) |

| | | | | |
|---|---|---|---|---|
| The percentages "% w/w" are expressed in relation to the total weight of each composition. In composition 1X, for 100 g of the composition, the composition comprises 15 g of Poloxamer 407, 85 g of bicarbonate buffer and xaliproden is solubilized at a concentration of 0,07 mg/ml. Compositions 1X, 2X and 3X are solutions. Composition 4X is a suspension and the percentages "% w/v" are expressed in relation to the total volume of the composition. | | | | |

The four lipid-based mucoadhesive solutions comprising paliroden are as follows:

**Table 5**

| | Composition 5P | Composition 6P | Composition 7P | Composition 8P |
|---|---|---|---|---|
| Plurol^{®} Oleique CC 497 | 100 % v/v | - | - | - |
| INTRALIPIDE 20% | - | 100 % v/v | - | - |
| Cremophor EL^{®} | - | - | **100** % v/v | - |
| Phosal^{®} 53 MCT | - | - | - | 100 % v/v |
| Micronized paliroden (fumarate salt) | 0.81 mg/mL | 1.5 mg/mL | 8.1 mg/mL | > 10 mg/mL |

| | | | | |
|---|---|---|---|---|
| The percentages "% v/v" are expressed in relation to the total volume of each composition. Compositions 5P, 6P, 7P and 8P are solutions. | | | | |

The four lipid-based mucoadhesive solutions comprising xaliproden are as follows:

**Table 6**

| | Composition 5X | Composition 6X | Composition 7X | Composition 8X |
|---|---|---|---|---|
| Plurol^{®} Oleique CC 497 | 100 % v/v | - | - | - |
| INTRALIPIDE 20% | - | 100 % v/v | - | - |
| Cremophor EL^{®} | - | - | 100 % v/v | - |
| Phosal^{®} 53 MCT | - | - | - | 100 % v/v |
| Micronized xaliproden (hydrochloride salt) | 2.4 mg/mL | 2.1 mg/mL | 6.4 mg/mL | > 10 mg/mL |

| | | | | |
|---|---|---|---|---|
| The percentages "% v/v" are expressed in relation to the total volume of each composition. Compositions 5X, 6X, 7X and 8X are solutions. | | | | |

### Results

The solubility of respectively paliroden and xaliproden in the compositions was measured. The results are presented in the following table:

**Table 7**

| | Solubility of paliroden (fumarate salt) (in mg/mL) | Solubility of xaliproden (hydrochloride salt) (in mg/mL) |
|---|---|---|
| Compositions 1 (1P and 1X) | 0.19 | 0.07 |
| Compositions 2 (2P and 2X) | 0.59 | 0.12 |
| Compositions 3 (3P and 3X) | 2.3 | 0.6 |
| Compositions 4 (4P and 4X) | 2.9 | 1.2 |
| Compositions 5 (5P and 5X) | 0.81 | 2.4 |
| Compositions 6 (6P and 6X) | 1.5 | 2.1 |
| Compositions 7 (7P and 7X) | 8.1 | 6.4 |
| Compositions 8 (8P and 8X) | >10 | >10 |

Therefore, the formulated thermoreversible gel compositions and lipid-based mucoadhesive solutions contained a significant portion of paliroden or xaliproden in solution, compared with a solubility of less than 0.1 mg/mL in water for both molecules. This makes it possible to expect that paliroden or xaliproden will be able to cross the round window membrane and enter the inner ear when administered in compositions 2P to 8P or 2X to 8X respectively.

Among the thermoreversible gel compositions, the highest solubilities of paliroden and xaliproden were obtained respectively in compositions 4P and 4X. These compositions 4P and 4X were shown to gel in less than 10 minutes at 37°C and are usable below 25°C for injection through a 26G needle for trans-tympanic injection.

Among the lipid-based mucoadhesive solutions, the highest solubilities of paliroden and xaliproden were obtained respectively in compositions 7P, 8P and 7X, 8X. Compositions 7P and 7X had viscosity values between 600 and 750 mPa.s (cP). Compositions 8P and 8X had viscosity values up to 5000 mPa.s (or cP). Compositions 7P, 8P, 7X and 8X were shown to be able to pass through a 26G needle for transtympanic injection.

### Example 3: Pharmacokinetic profiles of paliroden and xaliproden in the inner ear fluid of male Wistar rats following trans-tympanic injection in a lipid-based mucoadhesive solution

### Materials and Methods

Composition 7P and composition 7X as described in example 2 were administered to male Wistar rats (5 animals per active agent and timepoint), as described below.

At time T0, 30 µL of composition 7P were administered to 15 rats, in a single ear via trans-tympanic injection. The dose of paliroden fumarate that has been administered is thus of 243 µg in one ear of each rat.

At time T0, 30 µL of composition 7X were administered to 15 other rats, in a single ear via trans-tympanic injection. The dose of xaliproden hydrochloride that has been administered is thus of 181.2 µg in one ear of each rat.

For the 15 rats that have received composition 7P and for the 15 rats that have received composition 7X, plasmatic and inner ear expositions were determined via blood and cochlea terminal sampling in order to achieve lithium-heparin plasma and inner ear fluid preparation, at T+1 hour, T+24 hours and T+168 hours. Bioanalytical methods were validated in both matrices (lithium heparin plasma and inner ear fluid) with a range of 0.2 to 815 ng/mL for paliroden (fumarate salt) and 0.2 to 763 ng/mL for xaliproden (hydrochloride salt).

### Results

The results are presented in **figure 2** and in the following **tables 8 and 9:**

**Table 8 - Bioanalytical results for paliroden (fumarate salt) in plasma and inner ear fluids following single trans-tympanic injection of 243 µg of paliroden (fumarate salt) in a lipid-based mucoadhesive solution in one ear of each male Wistar rats at T0**

| Timepoints | Concentration of paliroden (fumarate salt) (ng/mL) | | | | | |
|---|---|---|---|---|---|---|
| | Plasma | | | IEF | | |
| | Mean | SD | RSD (%) | Mean | SD | RSD (%) |
| T0 + 1 hour | 0.9 | 0.1 | 11.1 | 5095.1 | 4650.2 | 91.3 |
| T0 + 24 hours | 1.7 | NA | NA | 3.2 | 1.7 | 53.1 |
| T0 + 168 hours | BLQ | NA | NA | BLQ | NA | NA |

| | | | | | | |
|---|---|---|---|---|---|---|
| SD = standard deviation. RSD = relative standard deviation. IEF = inner ear fluid. NA = not available. BLQ = below limit of quantification. | | | | | | |

**Table 9 - Bioanalytical results for xaliproden (hydrochloride salt) in plasma and inner ear fluids following single trans-tympanic injection of 181.2 µg of xaliproden (hydrochloride salt) in a lipid-based mucoadhesive solution in one ear of each male Wistar rats at T0**

| Timepoints | Concentration of xaliproden (hydrochloride salt) (ng/mL) | | | | | |
|---|---|---|---|---|---|---|
| | Plasma | | | IEF | | |
| | Mean | SD | RSD (%) | Mean | SD | RSD (%) |
| T0 + 1 hour | 4.4 | 1.3 | 29.5 | 2510.2 | 2367.6 | 94.3 |
| T0 + 24 hours | 5.2 | 4.0 | 84.6 | 74.3 | 97.2 | 130.8 |
| T0 + 168 hours | 0.5 | 0.3 | 60.0 | BLQ | NA | NA |

| | | | | | | |
|---|---|---|---|---|---|---|
| SD = standard deviation. RSD = relative standard deviation. IEF = inner ear fluid. NA = not available. BLQ = below limit of quantification. | | | | | | |

As shown in **tables 8 and 9** and in **figure 2****,** paliroden and xaliproden, when administered in a lipid-based mucoadhesive solution via a trans-tympanic injection, are able to cross the round window membrane and rapidly reach high concentrations in the inner ear fluid of approximately 5000 ng/mL and 2500 ng/mL respectively (approximately 10 µM and 5 µM in the IEF).

### Example 4: Pharmacokinetic profiles of paliroden and xaliproden in the inner ear fluid of male Wistar rats following trans-tympanic injection in a thermoreversible hydrogel composition

### Materials and Methods

Composition 4P and composition 4X as described in example 2 were administered to male Wistar rats (5 animals per active agent and timepoint), as described below.

At time T0, 30 µL of composition 4P were administered to 15 rats, in a single ear via trans-tympanic injection. The dose of paliroden fumarate that has been administered is thus of 300 µg in one ear of each rat.

At time T0, 30 µL of composition 4X were administered to 15 other rats, in a single ear via trans-tympanic injection. The dose of xaliproden hydrochloride that has been administered is thus of 150 µg in one ear of each rat.

For the 15 rats that have received composition 4P and for the 15 rats that have received composition 4X, plasmatic and inner ear expositions were determined via blood and cochlea terminal sampling in order to achieve lithium-heparin plasma and inner ear fluid preparation, at T+1 hour, T+24 hours and T+168 hours. Bioanalytical methods were validated in both matrices (lithium heparin plasma and inner ear fluid) with a range of 0.2 to 815 ng/mL for paliroden (fumarate salt) and 0.38 to 763 ng/mL for xaliproden (hydrochloride salt).

### Results

The results are presented in **figure 3** and in the following **tables 10 and 11:**

**Table 10 - Bioanalytical results for paliroden (fumarate salt) in plasma and inner ear fluids following single trans-tympanic injection of 300 µg of paliroden (fumarate salt) as a suspension in a thermoreversible hydrogel composition in one ear of each male Wistar rats at T0**

| Timepoints | Concentration of paliroden (fumarate salt) (ng/mL) | | | | | |
|---|---|---|---|---|---|---|
| | Plasma | | | IEF | | |
| | Mean | SD | RSD (%) | Mean | SD | RSD (%) |
| T0 + 1 hour | 1.0 | 0.16 | 16.0 | 5491.3 | 3027.3 | 55.1 |
| T0 + 24 hours | 0.4 | 0.1 | 25.0 | 1374.2 | 1422.9 | 103.5 |
| T0 + 168 hours | 0.2 | 0.05 | 25.0 | 686.8* | 1184.6 | 172.5 |

| | | | | | | |
|---|---|---|---|---|---|---|
| *n=4. SD = standard deviation. RSD = relative standard deviation. IEF = inner ear fluid. NA = not available. BLQ = below limit of quantification. | | | | | | |

**Table 11 - Bioanalytical results for xaliproden (hydrochloride salt) in plasma and inner ear fluids following single trans-tympanic injection of 150 µg of xaliproden (hydrochloride salt) as a suspension in a thermoreversible hydrogel composition in one ear of each male Wistar rats at T0**

| Timepoints | Concentration of xaliproden (hydrochloride salt) (ng/mL) | | | | | |
|---|---|---|---|---|---|---|
| | Plasma | | | IEF | | |
| | Mean | SD | RSD (%) | Mean | SD | RSD (%) |
| T0 + 1 hour | 4.9 | 4.5 | 92.5 | 5611.9* | 6276.9 | 111.9 |
| T0 + 24 hours | 9.2 | 6.6 | 71.3 | 108.4 | 126.4 | 116.7 |
| T0 + 168 hours | BLQ | NA | NA | 116.7 | 129.6 | 111 |

| | | | | | | |
|---|---|---|---|---|---|---|
| * n=4. SD = standard deviation. RSD = relative standard deviation. IEF = inner ear fluid. NA = not available. BLQ = below limit of quantification. | | | | | | |

As shown in **tables 10 and 11** and in **figure 3****,** paliroden and xaliproden, when administered as suspensions in a thermoreversible hydrogel composition via a trans-tympanic injection, are able to cross the round window membrane and rapidly reach high concentrations in the inner ear fluid of approximately 5000 ng/mL (approximately 10 µM in the IEF) and are sustainably released over 7 consecutive days with concentrations in the inner ear fluid at 7days post injection of approximately 600ng/ml (approximately 1.2µM) and 100ng/ml (approximately 0.2µM) for paliroden and xaliproden respectively.

### Example 5: Trans-tympanic administration (by injection between the eardrum and the round window by means of a needle which crosses the eardrum) of paliroden or xaliproden following acoustic trauma in male Wistar rats achieves hearing thresholds reduction

### Materials and Methods

Fourty-three male Wistar rats were selected and distributed to treatment and control groups, each group comprising between 6 to 9 rats at the start of the study. Randomization was performed using the ABR threshold in the left ear at 16 kHz as a criterium. The "sham" group comprises 7 rats, which received neither noise exposure nor trans-tympanic injection. The "untraumatized vehicle" group comprises 6 rats, which did not receive noise exposure but received a trans-tympanic injection of placebo (a composition comprising only Cremophor EL^{®}). The "traumatized" group comprises 9 rats, which received noise exposure only and no treatment. The "traumatized vehicle" group comprises 7 rats, which received noise exposure and a trans-tympanic injection of placebo (a composition comprising only Cremophor EL^{®}). The "traumatized paliroden" group comprises 7 rats, which received noise exposure and a trans-tympanic injection of a composition comprising paliroden (fumarate salt), corresponding to composition 7P described in Example 2. The "traumatized xaliproden" group comprises 7 rats, which received noise exposure and a trans-tympanic injection of a composition comprising xaliproden (hydrochloride salt), corresponding to composition 7X described in Example 2.

All animals (except sham group) received a bilateral noise exposure at a noise band of 8-16 kHz at 115 dB SPL RMS during 2 hours (from time T0 to time T0+2 hours).

30 µL of compositions comprising either paliroden or xaliproden as active agent were administered once in both ears of each rat of the treatment groups (30 µL per ear) via trans-tympanic administration by injection between the eardrum and the round window by means of a needle which crosses the eardrum, 4 hours after the start of the noise exposure (i.e. at T0+4 hours, thus 2 hours after the end of the noise exposure), under isoflurane anesthesia. The compositions correspond respectively to compositions 7P and 7X described in Example 2, i.e. they comprise only Cremophor EL^{®} as excipient and either paliroden (fumarate salt) or xaliproden (hydrochloride salt) as active agent. The dose of paliroden (fumarate salt) that is administered is 240 µg per ear of the rats belonging to the "traumatized paliroden" group (240 µg in the 30 µL that are injected via trans-tympanic route by injection between the eardrum and the round window by means of a needle which crosses the eardrum). The dose of xaliproden (hydrochloride salt) that is administered is 192 µg per ear of the rats belonging to the "traumatized xaliproden" group (192 µg in the 30 µL that are injected via trans-tympanic route by injection between the eardrum and the round window by means of a needle which crosses the eardrum).

To summarize, the chronology of the protocol was as follows:
- T0 - 1 day: ABR baseline assessment
- T0: Start of the noise exposure, trauma beginning
- T0 + 2 hours: end of the noise exposure, trauma end
- T0 + 4 hours: trans-tympanic injection in both ears of the rats belonging to treatment groups, the injected composition comprising either paliroden (fumarate salt) or xaliproden (hydrochloride salt) as active agent. 240 µg of paliroden (fumarate salt) were administered in each ear of the rats belonging to the "traumatized paliroden" group and 192 µg of xaliproden (hydrochloride salt) were administered in each ear of the rats belonging to the "traumatized xaliproden" group. In the "traumatized vehicle" group, animals received a 30µL Cremophor EL ^{®} injection between the eardrum and the round window by means of a needle which crosses the eardrum.
- T0 + 1 day: ABR assessment (n=6 to 14 ears)
- T0 + 7 days: ABR assessment (n=6 to 14 ears)

### Results

The results are presented in **figures 4** **and** **5****.**

The protocol used in this study (8-16 kHz sound at 115 dB SPL RMS, 2 hours, sound emitted in open field) induced hearing loss in Wistar rats after noise exposure as demonstrated in all traumatized groups by a significant increase of ABR thresholds at T0 + 1 day (see **figure 4****).** The hearing impairments were still observed at T0 + 7 days for the traumatized animals, with threshold shifts superior to 20 dB from 8 to 32 kHz (see **figure 4****).**

The trans-tympanic administration of the vehicle, alone without combination to noise-trauma, induced an increase of ABR thresholds in comparison to control animals (see **figure 4****).** Therefore, the groups treated with paliroden (fumarate salt) or xaliproden (hydrochloride salt) were compared to the traumatized vehicle group (and not traumatized untreated group, data not shown), in order to take into consideration the effect of the trans-tympanic injection of vehicle on the ABR thresholds.

Hearing improvement was observed in both paliroden and xaliproden treated groups, as shown in **figure 4** and **figure 5****.**

Paliroden at 240 µg/ear and xaliproden at 192 µg/ear exhibit a beneficial effect on hearing function in rats, already at one day following noise exposure. with ABR Threshold shifts reduction of approximately 10dB from 25 to 32 kHz in comparison to the traumatized vehicle group **(****Figure 4****).**

This beneficial effect on the hearing function in rats was maintained and extended at 7 days post-noise. Paliroden at 240 µg/ear and xaliproden at 192 µg/ear achieve ABR Threshold shifts reduction of approximately 15 dB from 25 to 32 kHz for xaliproden and approximately 15-20 dB from 16 to 32 kHz paliroden treatments in comparison to the traumatized vehicle group **(****Figure 4****).**

Overall, in addition to the hearing improvement observed at at T0 + 1 day, Paliroden at 240 µg/ear and xaliproden at 192 µg/ear allow an additional hearing recovery of approximately 5 to 10 dB from 32 to 25 kHz for xaliproden and from 5 to 20dB from 32 to 16kHz for paliroden treatments in comparison to the traumatized vehicle group between T0 + 7 days and T0 + 1 day **(****Figure 5****).**

### Example 6: Treatment of noise-induced synaptopathy in male Wistar rats with permanent hearing loss by immediate trans-tympanic administration (by injection between the eardrum and the round window by means of a needle which crosses the eardrum) of paliroden or xaliproden.

Fifty male Wistar rats were selected and distributed to treatment ("traumatized paliroden" group and "traumatized xaliproden" group) and control groups ("sham" group, "traumatized" group and "traumatized vehicle" group), each group comprising at least 9 rats at the end of the study. Randomization was performed using the ABR threshold in the left ear at 16 kHz as a criterium. The "sham" group consists in rats (n=11) that received neither noise exposure nor trans-tympanic injection. The "traumatized" group consists of rats (n=9) that received noise exposure only and no treatment. The "traumatized vehicle" group consists in rats (n=10) that received noise exposure and trans-tympanic injection in both ears of 30 µL of placebo (a composition comprising Poloxamer 407 (7,2%w/w), Poloxamer 188 (8%w/w) and Cremophor EL^{®} (20%w/w) in phosphate buffer pH=7.4, the percentages being expressed relative to the total weight of the composition) between the eardrum and the round window by means of a needle which crosses the eardrum, 4 hours after the start of the noise exposure (i.e. injection of placebo at T0+4 hours, thus 2 hours after the end of the noise exposure) and 8 days post-noise exposure, under isoflurane anesthesia. The "traumatized paliroden" group consists in rats (n=10) that received noise exposure and trans-tympanic injections of a composition comprising paliroden (fumarate salt), corresponding to composition 4P described in Example 2. The "traumatized xaliproden" group consists in rats (n=10) that received noise exposure and trans-tympanic injections of a composition comprising xaliproden (hydrochloride salt), corresponding to composition 4X described in Example 2.

All animals (except sham group) received a bilateral noise exposure at a noise band of 8-16 kHz at 110 dB SPL RMS during 2 hours (from time T0 to time T0+2 hours).

30 µL of compositions comprising either paliroden or xaliproden as active agent were administered once in both ears of each rat of the treatment groups (30 µL per ear) via trans-tympanic administration by injection between the eardrum and the round window by means of a needle which crosses the eardrum, 4 hours after the start of the noise exposure (i.e. at T0+4 hours, thus 2 hours after the end of the noise exposure) and 8 days post-noise exposure, under isoflurane anesthesia. The compositions correspond respectively to compositions 4P and 4X described in Example 2, i.e. they comprise a mixture of poloxamer 407, poloxamer 188 and Cremophor EL^{®} (7.2%w/w; 8%w/w and 20%w/w respectively, the percentages being expressed relative to the total weight of the composition) and either paliroden (fumarate salt) or xaliproden (hydrochloride salt) as active agent. The dose of paliroden (fumarate salt) that is administered is 300 µg per ear of the rats belonging to the "traumatized paliroden" group (300 µg in the 30 µL that are injected via trans-tympanic route by injection between the eardrum and the round window by means of a needle which crosses the eardrum). The dose of xaliproden (hydrochloride salt) that is administered is 150 µg per ear of the rats belonging to the "traumatized xaliproden" group (150 µg in the 30 µL that are injected via trans-tympanic route by injection between the eardrum and the round window by means of a needle which crosses the eardrum).

To summarize, the chronology of the protocol was as follows:
- T0 - 1 day: ABR/DPOAE baseline assessment
- T0: Start of the noise exposure, trauma beginning
- T0 + 2 hours: end of the noise exposure, trauma end
- T0 + 4 hours: trans-tympanic injection in both ears of the rats belonging to treatment groups and "traumatized vehicle" group, the injected composition comprising either paliroden (fumarate salt) or xaliproden (hydrochloride salt) as active agent (treatment groups) or a placebo ("traumatized vehicle" group). 300 µg of paliroden (fumarate salt) were administered in each ear of the rats belonging to the "traumatized paliroden" group and 150 µg of xaliproden (hydrochloride salt) were administered in each ear of the rats belonging to the "traumatized xaliproden" group. In the "traumatized vehicle" group, animals received 30µL of a mixture of poloxamer 407, poloxamer 188 and Cremophor EL^{®} (7.2%w/w; 8%w/w and 20%w/w respectively, the percentages being expressed relative to the total weight of the composition) by injection between the eardrum and the round window by means of a needle which crosses the eardrum.
- T0 + 1 day: ABR/DPOAE assessment (n=18-22 ears/group)
- T0 + 7 days: ABR assessment (n=18-22 ears/group)
- T0 + 8 days: Repetition of trans-tympanic injection in both ears of the rats belonging to treatment groups and "traumatized vehicle" group, with identical compositions comprising either paliroden (fumarate salt) or xaliproden (hydrochloride salt) as active agent and a mixture of poloxamer 407, poloxamer 188 and Cremophor EL^{®} (7,2%w/w ; 8%w/w and 20%w/w respectively, the percentages being expressed relative to the total weight of the composition) as vehicle control (treatment groups) or a placebo ("traumatized vehicle" group).
- T0 + 14 days: ABR/DPOAE assessment (n=18-22 ears/group)
- T0 + 21 days: ABR/DPOAE assessment (n=18-22 ears/group)
- T0 + 28 days: ABR/DPOAE assessment (n=18-22 ears/group)

At T+28DAYS, after DPOAE/ABR measures, 10 cochleae (all eight (8) left cochleae and two (2) right cochleae taken randomly) per group were rapidly sampled and immediately fixed by PFA perfusion (through the round and oval windows with PFA 4% after creating a small hole at the apex) in order to performed ribbon synapse immunostaining and analysis. Cochleae were fixed for 1 hour in a 4% PFA solution and decalcified in undiluted RDO (10 mL/cochlea for a minimum of 2 hours). The membranous and sensory spiral containing the organ of Corti are dissected out as a flat surface preparation under a dissecting microscope and immunolabelled with appropriate antibodies:
- Vglut3 (Vglut3-Guinea Pig IgG; #135 204, Synaptic systems and Anti-Guinea Pig IgG (H+L), #A21450; Thermo Fisher Scientific 647nm Far-Red) to stain inner hair cell bodies (vesicular glutamate transporter).
- Homer (Homer-Rabbit IgG; #GTX103278; Genetex and Anti-Rabbit IgG (H+L) #A11008 Thermo Fisher Scientific 568nm Red) to stain dendritic proteins of auditory nerve fibers connecting to IHC (post-synaptic).
- CtBP2 (CtBP2-Mouse-IgG1; #612044; BD Transduction Laboratories and AntiMouse IgG1, #A21121, Thermo Fisher Scientific 488 Green) to stain the ribeye protein as pre-synaptic marker in IHC.
- Phalloidin (Alexa Fluor^{™} Plus 405 Phalloidin; #A30104; Thermo Fisher Scientific 405 Blue).

Images were acquired on a confocal microscope and the slides were analyzed to i) evaluate by visual observation the presence of OHC (outer hair cells) and IHC (inner hair cells) (based on nuclei staining with CtBP2 and HC cell body staining with phalloidin) and ii) count the number of synaptic ribbons in selected inner hair cells in cochlea regions coding sound of selected frequency (16 kHz, 25 kHz and 32 kHz).

The number of pre- (CtBP2) and post-synaptic (Homer) labeled spots was counted using Imaris software in 3 dimensions for each image. Co-localization of pre- and post-synaptic elements was defined as a distance of maximum 1 µm between CtBP2- and Homer-labeled spots, using an in-house Matlab program to calculate the distances between CtBP2 and Homer labels from the x, y, and z coordinates.

### Results

The results are presented in **Figure 6****.**

The protocol used in this study (8-16 kHz sound at 110 dB SPL RMS, 2 hours, sound emitted in open field) induced permanent hearing loss in Wistar rats after noise exposure, materialized by residual ABR thresholds shifts and DPOAE amplitude reduction from baseline of approximately 20dB at T0 + 28 days in all traumatized animals (data not shown).

Xaliproden, Paliroden and vehicle treatment did not show effect on ABR thresholds, DPOAE amplitudes nor ABR Wave I amplitudes at T0 + 28 days in comparison to the traumatized animals either untreated or treated with the vehicle.

ABR wave I amplitudes measured at T0 + 28 days, mainly reflect the permanent hearing loss and cannot be used to evaluate the treatment of synaptopathy, defined as reduced of ABR wave I amplitudes in animals with normal hearing thresholds comparable to sham group, after noise exposure causing temporary ABR thresholds increase.

Therefore, synaptopathy was investigated by immunohistochemistry on noise-exposed cochlea versus sham cochlea, comparing the number of intact synapses (colocalizing pre- and post-synaptic elements) in traumatized animals with untraumatized control animals and focusing on basal cochlear regions coding higher frequencies (16 kHz, 25 kHz and 32 kHz) which are mostly affected by the acoustic trauma noise band (see **Figure 6****).**

Paliroden at 300 µg/ear and xaliproden at 150 µg/ear administered 2 hours and 8 days post-noise lead to an increase in the number of colocalized ribbons per IHC at 25kHz in comparison to the traumatized vehicle group **(****Figure 6C****).** Paliroden at 300 µg/ear was also found to exhibit similar effect at 32kHz.

Additionally, Paliroden at 300 µg/ear and xaliproden at 150 µg/ear administered 2 hours and 8 days post-noise lead to an increase in the number of pre-synaptic elements CtBP2 per IHC at 25kHz and 32kHz in comparison to the traumatized vehicle group **(****Figure 6A****).**

Overall, Paliroden at 300 µg/ear and xaliproden at 150 µg/ear administered 2 hours and 8 days post-noise tend to repair synapse loss in the inner hair cells of rats exposed to noise causing permanent hearing loss, in regions coding high frequency sounds. In human translation, this is expected to enhance the sound coding capabilities of patients and address their intelligibility deficit in noisy environments (hidden hearing loss).

### Example 7: Treatment of noise-induced synaptopathy in male Wistar rats with permanent hearing loss by delayed trans-tympanic administration (by injection between the eardrum and the round window by means of a needle which crosses the eardrum) of paliroden or xaliproden.

Forty male Wistar rats were selected and evenly distributed to treatment and control groups, each group comprising 8 rats at the end of the study. Randomization was performed using the ABR threshold in the left ear at 16 kHz as a criterium. The "sham" group consists in rats (n=8) that received neither noise exposure nor trans-tympanic injection. The "traumatized NaCl" group consists of rats (n=8) that received noise exposure only and trans-tympanic injections of a saline solution. The "traumatized vehicle" group consists in rats (n=8) that received noise exposure and trans-tympanic injections of placebo (a composition comprising Poloxamer 407 (7,2%w/w), Poloxamer 188 (8%w/w) and Cremophor EL^{®} (20%w/w) in phosphate buffer pH=7.4, the percentages being expressed relative to the total weight of the composition). The "traumatized paliroden" group consists in rats (n=8) that received noise exposure and trans-tympanic injections of a composition comprising paliroden (fumarate salt), corresponding to composition 4P described in Example 2. The "traumatized xaliproden" group consists in rats (n=10) that received noise exposure and a trans-tympanic injection of a composition comprising xaliproden (hydrochloride salt), corresponding to composition 4X described in Example 2.

All animals (except sham group) received a bilateral noise exposure at a noise band of 8-16 kHz at 110 dB SPL RMS during 2 hours (from time T0 to time T0+2 hours).

30 µL of a saline solution (0.9%w/w of NaCl in water) were administered once in both ears of each rat of the "traumatized NaCl" group (30 µL per ear) via trans-tympanic administration by injection between the eardrum and the round window by means of a needle which crosses the eardrum, 2 days and 9 days post noise exposure, under isoflurane anesthesia.

30 µL of the placebo (a composition comprising Poloxamer 407 (7,2%w/w), Poloxamer 188 (8%w/w) and Cremophor EL^{®} (20%w/w) in phosphate buffer pH=7.4, the percentages being expressed relative to the total weight of the composition) were administered once in both ears of each rat of the "traumatized vehicle" group (30 µL per ear) via trans-tympanic administration by injection between the eardrum and the round window by means of a needle which crosses the eardrum, 2 days and 9 days post noise exposure, under isoflurane anesthesia.

30 µL of compositions comprising either paliroden or xaliproden as active agent were administered once in both ears of each rat of the treatment groups (30 µL per ear) via trans-tympanic administration by injection between the eardrum and the round window by means of a needle which crosses the eardrum, 2 days and 9 days post noise exposure, under isoflurane anesthesia. The compositions correspond respectively to compositions 4P and 4X described in Example 2, i.e. they comprise a mixture of poloxamer 407, poloxamer 188 and Cremophor EL^{®} (7.2%w/w; 8%w/w and 20%w/w respectively, the percentages being expressed relative to the total weight of the composition) and either paliroden (fumarate salt) or xaliproden (hydrochloride salt) as active agent. The dose of paliroden (fumarate salt) that is administered is 300 µg per ear of the rats belonging to the "traumatized paliroden" group (300 µg in the 30 µL that are injected via trans-tympanic route by injection between the eardrum and the round window by means of a needle which crosses the eardrum). The dose of xaliproden (hydrochloride salt) that is administered is 150 µg per ear of the rats belonging to the "traumatized xaliproden" group (150 µg in the 30 µL that are injected via trans-tympanic route by injection between the eardrum and the round window by means of a needle which crosses the eardrum).

To summarize, the chronology of the protocol was as follows:
- T0 - 1 day: ABR/DPOAE baseline assessment
- T0: Start of the noise exposure, trauma beginning
- T0 + 1 day: ABR/DPOAE assessment (n=16 ears/group) and randomization based on hearing post-noise at 16kHz.
- T0 + 2 days and T + 9 days: trans-tympanic injection in both ears of the rats belonging to treatment groups, "traumatized NaCl" group and "traumatized vehicle" group, the injected composition comprising either paliroden (fumarate salt) or xaliproden (hydrochloride salt) as active agent (treatment groups) or a placebo ("traumatized vehicle" group) or a saline solution ("traumatized NaCl" group). 300 µg of paliroden (fumarate salt) were administered in each ear of the rats belonging to the "traumatized paliroden" group and 150 µg of xaliproden (hydrochloride salt) were administered in each ear of the rats belonging to the "traumatized xaliproden" group. In the "traumatized vehicle" group, animals received in both ears 30 µL of a mixture of poloxamer 407, poloxamer 188 and Cremophor EL^{®} (7.2%w/w; 8%w/w and 20%w/w respectively, the percentages being expressed relative to the total weight of the composition) by injection between the eardrum and the round window by means of a needle which crosses the eardrum. In the "traumatized NaCl" group, animals received 30 µL in both ears of a saline solution.
- T0 + 21 days: ABR/DPOAE assessment (n=16 ears/group)
- T0 + 28 days: ABR/DPOAE assessment (n=16 ears/group)

At T+28 days, after DPOAE/ABR measures, 10 cochlea (all eight (8) left cochleae and two (2) right cochleae taken randomly) per group were rapidly sampled and immediately fixed by PFA perfusion (through the round and oval windows with PFA 4% after creating a small hole at the apex) in order to performed ribbon synapse immunostaining and analysis. Cochleae were fixed for 1 hour in a 4% PFA solution and decalcified in undiluted RDO (10 mL/cochlea for a minimum of 2 hours). The membranous and sensory spiral containing the organ of Corti are dissected out as a flat surface preparation under a dissecting microscope and immunolabelled with appropriate antibodies:
- Vglut3 (Vglut3-Guinea Pig IgG; #135 204, Synaptic systems and Anti-Guinea Pig IgG (H+L), #A21450; Thermo Fisher Scientific 647nm Far-Red) to stain inner hair cell bodies (vesicular glutamate transporter).
- Homer (Homer-Rabbit IgG; #GTX103278; Genetex and Anti-Rabbit IgG (H+L) #A11008 Thermo Fisher Scientific 568nm Red) to stain dendritic proteins of auditory nerve fibers connecting to IHC (post-synaptic).
- CtBP2 (CtBP2-Mouse-IgG1; #612044; BD Transduction Laboratories and AntiMouse IgG1, #A21121, Thermo Fisher Scientific 488 Green) to stain the ribeye protein as pre-synaptic marker in IHC.
- Phalloidin (Alexa Fluor^{™} Plus 405 Phalloidin; #A30104; Thermo Fisher Scientific 405 Blue).

Images were acquired on a confocal microscope and the slides were analyzed to i) evaluate by visual observation the presence of OHC and IHC (based on nuclei staining with CtBP2 and HC cell body staining with phalloidin) and ii) count the number of synaptic ribbons in selected inner hair cells in cochlea regions coding sound of selected frequency (16 kHz, 25 kHz and 32 kHz).

The number of pre- (CtBP2) and post-synaptic (Homer) labeled spots was counted using Imaris software in 3 dimensions for each image. Co-localization of pre- and post-synaptic elements was defined as a distance of maximum 1 µm between CtBP2- and Homer-labeled spots, using an in-house Matlab program to calculate the distances between CtBP2 and Homer labels from the x, y, and z coordinates.

### Results

The results are presented in **Figure 7** **and** **Figure 8****.**

The protocol used in this study (8 kHz to 16 kHz sound at 110 dB SPL RMS, 2 hours, sound emitted in open field) induced permanent hearing loss in Wistar rats after noise exposure, materialized by residual ABR thresholds shifts and DPOAE amplitude reduction from baseline of approximately 20 dB at T0 + 28 days in all traumatized animals (data not shown).

Xaliproden, Paliroden and vehicle treatment did not show effect on ABR thresholds, DPOAE amplitudes nor ABR Wave I amplitudes at T0 + 28 days in comparison to the traumatized animals either untreated or treated with the vehicle.

ABR wave I amplitudes measured at T0 + 28 days, mainly reflect the permanent hearing loss and cannot be used to evaluate the treatment of synaptopathy, defined as reduced of ABR wave I amplitudes in animals with normal hearing thresholds comparable to sham group, after noise exposure causing temporary ABR thresholds increase. Indeed, the decrease in wave I amplitude is attributable to synaptopathy only in the context of a trauma with transient deafness. In the case of permanent deafness, even moderate permanent deafness, the wave I amplitude decrease also reflects this permanent deafness and not only the loss of synapses.

Therefore, synaptopathy was investigated by immunohistochemistry on noise-exposed cochlea versus sham cochlea, comparing the number of intact synapses (colocalizing pre- and post-synaptic elements) in traumatized animals with untraumatized control animals and focusing on basal cochlear regions coding higher frequencies (16 kHz, 25 kHz and 32 kHz) which are mostly affected by the acoustic trauma noise band (see **Figure 7****).**

Paliroden at 300 µg/ear and xaliproden at 150 µg/ear, administered 2 days and 9 days post-noise, lead to an increase in the number of colocalized ribbons per IHC at 25kHz in comparison to the traumatized vehicle and traumatized NaCl groups **(****Figure** 7C), which was found statistically significant for Paliroden at this frequency. Paliroden at 300 µg/ear was also found to exhibit similar effect at 16kHz.

Additionally, Paliroden at 300 µg/ear and xaliproden at 150 µg/ear administered 2 days and 9 days post-noise lead to an increase in the number of pre-synaptic and post-synaptic elements (CtBP2 and Homer respectively) per IHC at 25kHz in comparison to the traumatized vehicle and traumatized NaCl groups **(****Figure 7A and Figure 7B****).**

As shown in **Figure 8****,** Paliroden at 300 µg/ear and xaliproden at 150 µg/ear administered 2 hours and 8 days post-noise significantly repair synapse loss in the inner hair cells of rats exposed to noise causing permanent hearing loss, in regions coding high frequency sounds. Significantly more orphan pre and post-synaptic elements (green dots and red dots respectively) are found in traumatized animals treated with NaCl or vehicle than animals treated with Paliroden. Significantly more co-localized synaptic elements, leading to intact and functional synapses, are therefore observed upon Paliroden and Xaliproden treatments. In human translation, this is expected to enhance the sound coding capabilities of patients and address their intelligibility deficit in noisy environments (hidden hearing loss).

### Example 8: Treatment of noise-induced synaptopathy in male CBA mice with persistent sound-coding deficiencies (hidden hearing loss) by immediate trans-tympanic administration (by injection between the eardrum and the round window by means of a needle which crosses the eardrum) of paliroden.

Twelve male CBA/CaJ mice were selected and evenly distributed to treatment and control groups, each group comprising 4 mice at the end of the study. Randomization was performed using the ABR threshold in the left ear at 16 kHz as a criterium. The "sham" group consists in mice (n=4) that received neither noise exposure nor trans-tympanic injection. The "Traumatized" group consists in mice (n=4) that received noise exposure only. The "traumatized paliroden" group consists in mice (n=4) that received noise exposure and trans-tympanic injections of a composition comprising paliroden (fumarate salt), corresponding to composition 4P described in Example 2.

All animals (except sham group) received a bilateral noise exposure at a noise band of 8-16 kHz at 95 dB SPL RMS during 2 hours (from time T0 to time T0+2 hours) in awake conditions.

Five microliters of a composition comprising paliroden as active agent were administered once in both ears of each mice of the treatment groups (5 µL per ear) via trans-tympanic administration by injection between the eardrum and the round window by means of a needle which crosses the eardrum, 24 hours post noise exposure, under isoflurane anesthesia. The composition corresponds to composition 4P described in Example 2, i.e. it comprises a mixture of poloxamer 407, poloxamer 188 and Cremophor EL^{®} (7.2%w/w; 8%w/w and 20%w/w respectively, the percentages being expressed relative to the total weight of the composition) with paliroden (fumarate salt). The dose of paliroden (fumarate salt) that is administered is 50 µg per ear of the mice belonging to the "traumatized paliroden" group (50 µg in the 5 µL that are injected via trans-tympanic route by injection between the eardrum and the round window by means of a needle which crosses the eardrum).

To summarize, the chronology of the protocol was as follows:
- T0 - 1 day: ABR/DPOAE baseline assessment
- T0: Start of the noise exposure, trauma beginning in awake conditions
- T0 + 1 day: ABR/DPOAE assessment (n=8 ears/group) and randomization based on hearing post-noise at 16kHz. Trans-tympanic injection in both ears of the mice belonging to Paliroden treatment group,
- T0 + 35 days: ABR/DPOAE assessment (n=8 ears/group)

At T+35 days, after DPOAE/ABR measures, all cochlea (four (4) left cochleae and four (4) right cochleae) per group were rapidly sampled and immediately fixed by PFA perfusion (through the round and oval windows with PFA 4% after creating a small hole at the apex) in order to performed ribbon synapse immunostaining and analysis. Cochleae were fixed for 1 hour in a 4% PFA solution and decalcified in undiluted RDO (10 mL/cochlea for a minimum of 2 hours). The membranous and sensory spiral containing the organ of Corti are dissected out as a flat surface preparation under a dissecting microscope and immunolabelled with appropriate antibodies:
- Vglut3 (Vglut3-Guinea Pig IgG; #135 204, Synaptic systems and Anti-Guinea Pig IgG (H+L), #A21450; Thermo Fisher Scientific 647nm Far-Red) to stain inner hair cell bodies (vesicular glutamate transporter).
- Homer (Homer-Rabbit IgG; #GTX103278; Genetex and Anti-Rabbit IgG (H+L) #A11008 Thermo Fisher Scientific 568nm Red) to stain dendritic proteins of auditory nerve fibers connecting to IHC (post-synaptic).
- CtBP2 (CtBP2-Mouse-IgG1; #612044; BD Transduction Laboratories and AntiMouse IgG1, #A21121, Thermo Fisher Scientific 488 Green) to stain the ribeye protein as pre-synaptic marker in IHC.
- Phalloidin (Alexa Fluor^{™} Plus 405 Phalloidin; #A30104; Thermo Fisher Scientific 405 Blue).

Images were acquired on a confocal microscope and the slides were analyzed to i) evaluate by visual observation the presence of OHC and IHC (based on nuclei staining with CtBP2 and HC cell body staining with phalloidin) and ii) count the number of synaptic ribbons in selected inner hair cells in cochlea regions coding sound of selected frequency (25, 32 and 45kHz).

The number of pre- (CtBP2) and post-synaptic (Homer) labeled spots was counted using Imaris software in 3 dimensions for each image. Co-localization of pre- and post-synaptic elements was defined as a distance of maximum 1 µm between CtBP2- and Homer-labeled spots, using an in-house Matlab program to calculate the distances between CtBP2 and Homer labels from the x, y, and z coordinates.

### Results

The results are presented in **Figure 9****,** **Figure 10****,** **Figure 11****,** **Figure 12** **and** **Figure 13****.**

The protocol used in this study (awake noise trauma at 8-16 kHz, 95 dB SPL RMS, 2 hours, sound emitted in open field) induced temporary hearing loss in CBA/CaJ mice after noise exposure, materialized by temporary ABR thresholds increase and DPOAE amplitude reduction of at least 20dB from baseline at T0 + 1 day **(****Figure 9A** and **Figure 10A****),** with complete recovery observed at T0 + 35 days and ABR thresholds as well as DPOAE amplitude returning to normal values identical to those observed in the sham group (less than 10dB difference) **(****Figure 9B** **and** **Figure 10B****).**

In such model of temporary hearing loss, synaptopathy is characterized by a persistent significant decrease of ABR waves I at T0+35 days observed in the traumatized groups (Group 2) in comparison to the sham group (Group 1), as shown in **Figure 11A to Figure 11E****.** At 25, 32, 40 and 45kHz, traumatized untreated mice exhibit an immediate ABR wave I amplitude reduction 1 day post-noise exposure, ranging from 50 to 75% reduction in comparison to untraumatized control mice (sham group). At T0+35 days, these ABR Wave I amplitude decrease were found persistent, in the range of 25% to 50% showing very limited spontaneous recovery.

Paliroden treatment, occurring immediately after auditory evaluation one day post-noise exposure, led to complete recovery of the ABR Wave I amplitudes 5 weeks post-noise at all evaluated frequencies (16 kHz, 25 kHz, 32 kHz, 40 kHz, 45 kHz). Indeed, ABR Wave I amplitudes in traumatized mice treated with paliroden were found comparable to untraumatized control mice (sham group), at all frequencies.

In a subset analysis (displayed in **Figure 12** and **Figure 13****),** corresponding to only animals complying with acceptance criteria for noise exposure (i.e. at least 20 dB increase in ABR thresholds one day post-noise) and control normal conditions (sham should not exhibit ABR thresholds increase higher than 20dB in comparison to baseline level), the ABR Wave I amplitude recovery achieved by Paliroden treatment was found statistically significant at frequencies of 25 kHz **(****Figure 12A****),** 32 kHz **(****Figure 12B****),** 40 kHz **(****Figure 12C****)** and 45 kHz **(****Figure 12D****),** in comparison to traumatized untreated mice (p values < 0.001).

Additionally, in this identical subset analysis group, this finding was correlated with a significant decrease in the number of pre-synaptic and post-synaptic elements (CtBP2 and Homer respectively) per IHC at 32kHz in traumatized mice compared to control mice (Sham) (p value < 0.01; **Figure 13****).** Paliroden at 50 µg/ear administered one day post-noise tended to increase the number of pre-synaptic and post-synaptic elements (CtBP2 and Homer respectively) per IHC at 32kHz in comparison to the traumatized untreated groups **(****Figure 13A and Figure 13B****),** resulting in an increase as well as of colocalized ribbons per IHC representing functional synapses at 32kHz **(****Figure 13C****).** At frequencies ranging from 25kHz to 40kHz, the number of colocalized ribbons per IHC obtained in Paliroden treated mice were found intermediate between traumatized and control mice, and not statistically different to values obtained for control mice (sham).

### Example 9: Treatment of noise-induced synaptopathy in male CBA mice with persistent sound-coding deficiencies (hidden hearing loss) by immediate trans-tympanic administration (by injection between the eardrum and the round window by means of a needle which crosses the eardrum) of paliroden or xaliproden.

In another on-going experiment to complete the above described example 8, seventeen additional male CBA/CaJ mice are evenly distributed to treatment and control groups. Randomization is performed using the ABR threshold in the left ear at 16 kHz as a criterium. The "sham" group consists in mice (n=5) that received neither noise exposure nor trans-tympanic injection. The "Traumatized" group consists in mice (n=4) that receive noise exposure only. The "traumatized paliroden" group consists in mice (n=3) that receive noise exposure and trans-tympanic injections of a composition comprising paliroden (fumarate salt), corresponding to composition 4P described in Example 2. The "traumatized xaliproden" group consists in mice (n=5) that receive noise exposure and trans-tympanic injections of a composition comprising xaliproden (hydrochloride salt), corresponding to composition 4X described in Example 2.

Identical procedure to those of Example 8 are scheduled on these animals in order to demonstrate similar findings achieved by Xaliproden treatment. The expected results are that both Paliroden and Xaliproden treatment are able to restore ABR wave I amplitudes to normal levels (untraumatized animals), 5 weeks post-noise exposure causing temporary hearing loss with persistent sound coding deficiencies due to loss of afferent ribbon synapses at inner hair cell level. As shown for Paliroden, it is expected that Xaliproden repairs ribbon synapses of IHC in cochlea regions coding high frequencies (> 25 kHz).

In human translation, Paliroden and Xaliproden treatment are expected to address hidden hearing loss by increasing the sound coding capabilities of patients with normal or close to normal audiograms, with intelligibility deficit in noisy environments associated to auditory synapse loss.

## Claims

1. A compound of formula (I) or a pharmaceutically acceptable salt and/or solvate thereof, for use in the treatment of a hearing disease in a subject, wherein formula (I) is: 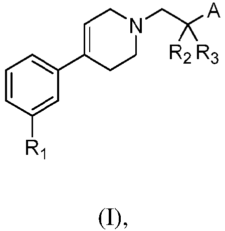 wherein
R₁ is a halogen atom, a CF₃ group, a (C₁-C₄)alkyl group or a (C₁-C₄)alkoxy group;
R₂ and R₃ are independently a hydrogen atom or a (C₁-C₃)alkyl group; and
A is:
- a phenyl radical substituted by a substituent X, wherein X is:
(a) a group selected from (C₃-C₆)cycloalkyl, (C₃-C₆)cycloalkylmethyl, (C₃-C₆)cycloalkoxy, (C₃-C₆)cycloalkylamino and cyclohexenyl; or
(b) a group selected from phenyl, phenylmethyl, phenylcarbonyl, phenoxy, phenylamino, N-(C₁-C₃)alkyl-phenylamino, phenylthio, phenylsulfinyl and phenylsulfonyl; or
- a 1-naphthyl or 2-naphthyl radical, either non-substituted or substituted in the 5-, 6-, 7- and/or 8-positions by one or two hydroxyl groups, one or two (C₁-C₄)alkoxy groups or a 6,7-methylenedioxy group.

2. The compound for use according to claim **1,** wherein R₁ is a CF₃ group.

3. The compound for use according to claim **1** or **2,** wherein at least one of R₂ and R₃ is a hydrogen atom, preferably R₂ and R₃ are each a hydrogen atom.

4. The compound for use according to any one of claims **1** to **3,** wherein A is a biphenyl radical or a non-substituted 2-naphthyl radical.

5. The compound for use according to any one of claims **1** to **4,** wherein the salt is a hydrochloride salt or a fumarate salt of the compound of formula (I).

6. The compound for use according to any one of claims **1** to **5,** wherein said compound is administered to the subject by oral route or by trans-tympanic route, preferably said compound is administered to the subject by oral route or by trans-tympanic route wherein the administration by trans-tympanic route consists in an injection between the eardrum and the round window by means of a needle which crosses the eardrum, more preferably the compound is administered to the subject by trans-tympanic route wherein the administration by trans-tympanic route consists in an injection between the eardrum and the round window by means of a needle which crosses the eardrum.

7. The compound for use according to any one of claims **1** to **6,** wherein said compound is administered by trans-tympanic route at a dose ranging from 10 µg to 400 mg, between once a month and once every 12 months, preferably said administration by trans-tympanic route consists in an injection between the eardrum and the round window by means of a needle which crosses the eardrum.

8. The compound for use according to any one of claims **1** to **7,** wherein the hearing disease is selected from the group consisting of unilateral or bilateral hearing loss, unilateral or bilateral tinnitus, unilateral or bilateral hyperacusis, hidden hearing loss such as cochlear synaptopathy associated with a normal threshold, speech-intelligibility deficit, unilateral or bilateral temporary auditory threshold shift, central auditory processing disorder, unilateral or bilateral auditory recruitment, acoustic neuroma, single sided deafness, excitotoxicity, ototoxicity such as drug-induced ototoxicity and any combination thereof,
preferably the hearing disease is selected from the group consisting of unilateral and bilateral hearing loss such as a unilateral and bilateral sensorineural hearing loss, unilateral or bilateral tinnitus, unilateral or bilateral hyperacusis, hidden hearing loss such as cochlear synaptopathy associated with a normal threshold, and speech-intelligibility deficit,
more preferably the hearing disease is a unilateral or bilateral sensorineural hearing loss selected from the group consisting of unilateral or bilateral noise-induced sensorineural hearing loss, unilateral or bilateral inflammation-induced sensorineural hearing loss, unilateral or bilateral sudden idiopathic sensorineural hearing loss, unilateral or bilateral ototoxic chemicals-induced sensorineural hearing loss and unilateral or bilateral aged-induced sensorineural hearing loss such as presbycusis,
even more preferably the hearing disease is a unilateral or bilateral sensorineural hearing loss selected from the group consisting of unilateral or bilateral noise-induced sensorineural hearing loss and unilateral or bilateral aged-induced sensorineural hearing loss.

9. The compound for use according to any one of claims **1** to **8,** wherein the hearing disease is a unilateral or bilateral inflammation-induced sensorineural hearing loss caused by a chronic inflammatory disease, preferably said chronic inflammatory disease is selected from the group consisting of diabetes, chronic kidney disease, inflammatory bowel diseases and rheumatoid arthritis.

10. The compound for use according to any one of claims **1** to **9,** wherein the hearing disease is a unilateral or bilateral hearing loss with a tonal audiometric threshold above 30 dB at 3 contiguous frequencies optionally associated with unilateral or bilateral tinnitus, preferably the hearing disease is a unilateral or bilateral hearing loss with a tonal audiometric threshold from 30 dB to 70 dB at 3 contiguous frequencies optionally associated with unilateral or bilateral tinnitus, the tonal audiometric threshold being measured by auditory brainstem response or pure-tone audiometry.

11. The compound for use according to any one of claims **1** to **10,** wherein said subject is wearing at least one hearing device.

12. The compound for use according to any one of claims **1** to **11,** wherein the compound of formula (I) or a pharmaceutically acceptable salt and/or solvate thereof is administered to the subject during a cochlear surgery, for example during a cochlear implant surgery.

13. The compound for use according to any one of claims **1** to **12,** wherein said subject is a mammalian animal with an eardrum, preferably said mammalian animal with an eardrum is selected from the group consisting of a human, a cat, a dog and a non-human primate animal, more preferably said mammalian animal with an eardrum is a dog.

14. A pharmaceutical transtympanic composition comprising a compound of formula (I) or a pharmaceutically acceptable salt and/or solvate thereof as defined in any one of claims **1** to **5** and a combination of a mixture of polyoxyethylated triglycerides and poloxamers, said pharmaceutical composition being a thermoreversible gel.

15. A pharmaceutical composition for use in the treatment of a hearing disease in a subject, said pharmaceutical composition comprising a compound of formula (I) or a pharmaceutically acceptable salt and/or solvate thereof according to any one of claims **1** to **5** and at least one pharmaceutically acceptable excipient, preferably said hearing disease being as defined in any one of claims **8** to **10.**

## Patentansprüche

1. Eine Verbindung mit der Formel (I) oder ein pharmazeutisch akzeptables Salz und/oder Solvat davon, zur Verwendung bei der Behandlung einer Hörerkrankung bei einem Probanden, wobei Formel (I) Folgendes ist: 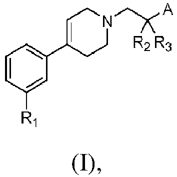 wobei
R₁ ein Halogenatom, eine CF₃-Gruppe, eine (C₁-C₄)-Alkylgruppe oder eine (C₁-C₄)-Alkoxygruppe ist;
R₂ und R₃ unabhängig voneinander ein Wasserstoffatom oder eine (C₁-C₃)-Alkylgruppe sind; und
A Folgendes ist:
- ein durch einen Substituenten X substituiertes Phenylradikal, wobei X Folgendes ist:
(a) eine Gruppe ausgewählt aus (C₃-C₆)-Cycloalkyl, (C₃-C₆)-Cycloalkylmethyl, (C₃-C₆)-Cycloalkoxy, (C₃-C₆)-Cycloalkylamino und Cyclohexenyl; oder
(b) eine Gruppe ausgewählt aus Phenyl, Phenylmethyl, Phenylcarbonyl, Phenoxy, Phenylamino, N-(C₁-C₃)-Alkylphenylamino, Phenylthio, Phenylsulfinyl und Phenylsulfonyl; oder
- ein 1-Naphtyl- oder 2-Naphtyl-Radikal, entweder nicht substituiert oder substituiert in den 5-, 6-, 7- und/oder 8-Positionen durch eine oder zwei Hydroxylgruppen, eine oder zwei (C₁-C₄)-Alkoxygruppen oder eine 6,7-Methylendioxygruppe.

2. Verbindung zur Verwendung nach Anspruch **1,** wobei R₁ eine CF₃-Gruppe ist.

3. Verbindung zur Verwendung nach Anspruch **1** oder **2,** wobei mindestens eines von R₂ und R₃ ein Wasserstoffatom ist, vorzugsweise R₂ und R₃ jeweils ein Wasserstoffatom sind.

4. Verbindung zur Verwendung nach einem der Ansprüche **1** bis **3,** wobei A ein Biphenylradikal oder ein nicht substituiertes 2-Naphtylradikal ist.

5. Verbindung zur Verwendung nach einem der Ansprüche **1** bis **4,** wobei das Salz ein Hydrochloridsalz oder ein Fumaratsalz der Verbindung der Formel (I) ist.

6. Verbindung zur Verwendung nach einem der Ansprüche **1** bis **5,** wobei die Verbindung dem Probanden oral oder über den transtympanischen Weg verabreicht wird, vorzugsweise die Verbindung dem Probanden oral oder über den transtympanischen Weg verabreicht wird, wobei die Verabreichung über den transtympanischen Weg in einer Injektion zwischen dem Trommelfell und dem runden Fenster mittels einer Nadel besteht, die das Trommelfell kreuzt, bevorzugter wird die Verbindung dem Probanden über den transtympanischen Weg verabreicht, wobei die Verabreichung über den transtympanischen Weg in einer Injektion zwischen dem Trommelfell und dem runden Fenster mittels einer Nadel besteht, die das Trommelfell kreuzt.

7. Verbindung zur Verwendung nach einem der Ansprüche **1** bis **6,** wobei die Verbindung in einer Dosis von 10 µg bis 400 mg zwischen einmal monatlich und einmal alle 12 Monate transtympanisch verabreicht wird, vorzugsweise besteht die Verabreichung über den transtympanischen Weg in einer Injektion zwischen dem Trommelfell und dem runden Fenster mittels einer Nadel, die das Trommelfell kreuzt.

8. Verbindung zur Verwendung nach einem der Ansprüche **1** bis **7,** wobei die Hörerkrankung aus der Gruppe ausgewählt ist, die aus einseitigem oder beidseitigem Hörverlust, einseitigem oder beidseitigem Tinnitus, einseitigem oder beidseitigem Hyperakusis besteht, versteckter Hörverlust, wie z. B. cochleäre Synaptopathie in Verbindung mit einer normalen Hörschwelle, Sprachverständlichkeitsdefizit, einseitige oder beidseitige vorübergehende Hörschwellenverschiebung, Störung der zentralen Hörverarbeitung, unilaterales oder bilaterales akustisches Recruitment, akustisches Neurom, einseitige Taubheit, Exzitotoxizität, Ototoxizität wie medikamenteninduzierte Ototoxizität und jede Kombination davon,
vorzugsweise die Hörerkrankung aus der Gruppe ausgewählt wird, die aus einem einseitigen und bilateralen Hörverlust wie einem einseitigen und bilateralen sensorineuralen Hörverlust, einem einseitigen oder bilateralen Tinnitus, einer einseitigen oder bilateralen Hyperakusis, einem versteckten Hörverlust wie einer mit einer normalen Schwelle assoziierten Cochleasynaptopathie und einem Sprachverständlichkeitsdefizit besteht, bevorzugter handelt es sich bei der Hörerkrankung um einen einseitigen oder bilateralen sensorineuralen Hörverlust, der aus der Gruppe besteht, die aus einem einseitigen oder bilateralen geräuschinduzierten sensorineuralen Hörverlust, einem einseitigen oder bilateralen entzündungsinduzierten sensorineuralen Hörverlust, einem einseitigen oder bilateralen plötzlichen idiopathischen sensorineuralen Hörverlust, einem einseitigen oder bilateralen ototoxisch-chemisch induzierten sensorineuralen Hörverlust und einem einseitigen oder bilateralen altersinduzierten sensorineuralen Hörverlust wie Presbykus besteht,
noch vorzugsweise handelt es sich bei der Hörerkrankung um einen einseitigen oder beidseitigen sensorineuralen Hörverlust, der aus der Gruppe bestehend aus einem einseitigen oder beidseitigen geräuschinduzierten sensorineuralen Hörverlust und einem einseitigen oder beidseitigen altersinduzierten sensorineuralen Hörverlust ausgewählt wird.

9. Verbindung zur Verwendung nach einem der Ansprüche **1** bis **8,** wobei die Gehörerkrankung ein einseitiger oder beidseitig entzündungsinduzierter sensorineuraler Hörverlust ist, der durch eine chronische entzündliche Erkrankung verursacht wird, vorzugsweise ist die chronische entzündliche Erkrankung aus der Gruppe ausgewählt, die aus Diabetes, chronischer Nierenerkrankung, entzündlichen Darmerkrankungen und rheumatoider Arthritis besteht.

10. Verbindung zur Verwendung nach einem der Ansprüche 1 bis **9,** wobei die Hörerkrankung ein einseitiger oder beidseitiger Hörverlust mit einer tonalen audiometrischen Schwelle von über 30 dB bei 3 zusammenhängenden Frequenzen ist, die optional mit einem einseitigen oder beidseitigen Tinnitus assoziiert ist, vorzugsweise die Hörerkrankung ein einseitiger oder beidseitiger Hörverlust mit einer tonalen audiometrischen Schwelle von 30 dB bis 70 dB bei 3 zusammenhängenden Frequenzen ist, die optional mit einem einseitigen oder beidseitigen Tinnitus assoziiert ist, die tonale audiometrische Schwelle wird durch auditive Hirnstammantwort oder Reintonaudiometrie gemessen.

11. Verbindung zur Verwendung nach einem der Ansprüche **1** bis **10,** wobei der Proband mindestens ein Hörgerät trägt.

12. Verbindung zur Verwendung nach einem der Ansprüche **1** bis **11,** wobei die Verbindung der Formel (I) oder ein pharmazeutisch akzeptables Salz und/oder dessen Solvat dem Probanden während einer Cochlea-Operation verabreicht wird, z. B. während einer Cochlea-Implantationsoperation.

13. Verbindung zur Verwendung nach einem der Ansprüche **1** bis **12,** wobei der Proband ein Säugetier mit einem Trommelfell ist, vorzugsweise das Säugetier mit einem Trommelfell aus der Gruppe ausgewählt ist, die aus einem Menschen, einer Katze, einem Hund und einem nichtmenschlichen Primaten besteht, bevorzugter ist das Säugetier mit einem Trommelfell ein Hund.

14. Eine pharmazeutische transtympanische Zusammensetzung, die eine Verbindung der Formel (I) oder ein pharmazeutisch akzeptables Salz und/oder Solvat davon, wie in einem der Ansprüche **1** bis **5** definiert, und eine Kombination aus einer Mischung aus polyoxyethylierten Triglyceriden und Poloxameren, wobei die pharmazeutische Zusammensetzung ein thermoreversibles Gel ist.

15. Pharmazeutische Zusammensetzung zur Verwendung bei der Behandlung einer Hörerkrankung bei einem Probanden, wobei die pharmazeutische Zusammensetzung eine Verbindung der Formel (I) oder ein pharmazeutisch akzeptables Salz und/oder Solvat davon nach einem der Ansprüche **1** bis **5** und mindestens einen pharmazeutisch akzeptablen Hilfsstoff, wobei vorzugsweise die Hörerkrankung wie in einem der Ansprüche **8** bis **10** definiert ist.

## Revendications

1. Composé de formule (I) ou un sel et/ou un solvate pharmaceutiquement acceptable de celui-ci, pour utilisation dans le traitement d'une maladie auditive chez un sujet, dans lequel la formule (I) est : 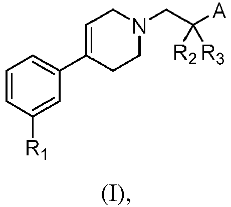 dans laquelle
R₁ est un atome d'halogène, un groupe CF₃, un groupe (C₁-C₄) alkyle ou un groupe (C₁-C₄) alcoxy;
R₂ et R₃ sont indépendamment un atome d'hydrogène ou un groupe (C₁-C₃) alkyle ; et
A est :
- un radical phényle substitué par un substituant X, dans lequel X est :
(a) un groupe choisi parmi : (C₃-C₆) cycloalkyle, (C₃-C₆) cycloalkylméthyle, (C₃-C₆) cycloalkoxy, (C₃-C₆) cycloalkylamino et cyclohexényle ; ou
(b) un groupe choisi parmi : phényle, phénylméthyle, phénylcarbonyle, phénoxy, phénylamino, N-(C₁-C₃)alkyl-phénylamino, phénylthio, phénylsulfinyle et phénylsulfonyle ; ou
- un radical 1-naphtyle ou 2-naphtyle, soit non substitué soit substitué en position 5, 6, 7 et/ou 8 par un ou deux groupes hydroxyle, un ou deux groupes (C₁-C₄) alcoxy ou un groupe 6,7-méthylènedioxy.

2. Composé pour utilisation selon la revendication **1,** dans lequel R₁ est un groupe CF₃.

3. Composé pour utilisation selon la revendication **1** ou **2,** dans lequel au moins un de R₂ et R₃ est un atome d'hydrogène, de préférence R₂ et R₃ sont chacun un atome d'hydrogène.

4. Composé pour utilisation selon l'une quelconque des revendications **1** à **3,** dans lequel A est un radical biphényle ou un radical 2-naphtyle non substitué.

5. Composé pour utilisation selon l'une quelconque des revendications **1** à **4,** dans lequel le sel est un sel de chlorhydrate ou un sel de fumarate du composé de formule (I).

6. Composé pour utilisation selon l'une quelconque des revendications **1** à **5,** dans lequel ledit composé est administré au sujet par voie orale ou par voie trans-tympanique, de préférence ledit composé est administré au sujet par voie orale ou par voie trans-tympanique dans laquelle l'administration par voie trans-tympanique consiste en une injection entre le tympan et la fenêtre ronde au moyen d'une aiguille qui traverse le tympan, plus préférentiellement, le composé est administré au sujet par voie trans-tympanique dans laquelle l'administration par voie trans-tympanique consiste en une injection entre le tympan et la fenêtre ronde au moyen d'une aiguille qui traverse le tympan.

7. Composé pour utilisation selon l'une quelconque des revendications **1** à **6,** dans lequel ledit composé est administré par voie trans-tympanique à une dose allant de 10 µg à 400 mg, entre une fois par mois et une fois tous les 12 mois, de préférence ladite administration par voie trans-tympanique consiste en une injection entre le tympan et la fenêtre ronde au moyen d'une aiguille qui traverse le tympan.

8. Composé pour utilisation selon l'une quelconque des revendications **1** à **7,** dans lequel la maladie auditive est choisie dans le groupe constitué par la perte d'audition unilatérale ou bilatérale, acouphène unilatéral ou bilatéral, hyperacousie unilatérale ou bilatérale, perte d'audition cachée telle que synaptopathie cochléaire associée à un seuil normal, déficit d'intelligibilité de la parole, décalage temporaire unilatéral ou bilatéral du seuil auditif, trouble du traitement auditif central, recrutement auditif unilatéral ou bilatéral, neurinome acoustique, surdité unilatérale, excitotoxicité, ototoxicité telle que l'ototoxicité induite par médicament et toute combinaison de ceux-ci,
préférentiellement, la maladie auditive est choisie dans le groupe constitué par la perte d'audition unilatérale et bilatérale telle que la perte d'audition neurosensorielle unilatérale et bilatérale, acouphène unilatéral ou bilatéral, hyperacousie unilatérale ou bilatérale, perte d'audition cachée telle qu'une synaptopathie cochléaire associée à un seuil normal, et déficit d'intelligibilité de la parole,
plus préférentiellement, la maladie auditive est une perte d'audition neurosensorielle unilatérale ou bilatérale choisie dans le groupe constitué par la perte d'audition neurosensorielle unilatérale ou bilatérale induite par le bruit, la perte d'audition neurosensorielle unilatérale ou bilatérale induite par l'inflammation, la perte d'audition neurosensorielle idiopathique soudaine unilatérale ou bilatérale, la perte d'audition neurosensorielle unilatérale ou bilatérale induite par des produits chimiques ototoxiques et la perte d'audition neurosensorielle unilatérale ou bilatérale induite par l'âge telle que la presbyacousie,
encore plus préférentiellement, la maladie auditive est une perte d'audition neurosensorielle unilatérale ou bilatérale choisie dans le groupe constitué par la perte d'audition neurosensorielle unilatérale ou bilatérale induite par le bruit et la perte d'audition neurosensorielle unilatérale ou bilatérale induite par l'âge.

9. Composé pour utilisation selon l'une quelconque des revendications **1** à **8,** dans lequel la maladie auditive est une perte d'audition neurosensorielle unilatérale ou bilatérale induite par l'inflammation et causée par une maladie inflammatoire chronique, de préférence ladite maladie inflammatoire chronique est choisie dans le groupe constitué par le diabète, les maladies rénales chroniques, les maladies intestinales inflammatoires et l'arthrite rhumatoïde.

10. Composé pour utilisation selon l'une quelconque des revendications **1** à **9,** dans lequel la maladie auditive est une perte d'audition unilatérale ou bilatérale avec un seuil audiométrique tonal supérieur à 30 dB à 3 fréquences contiguës, optionnellement associée à un acouphène unilatéral ou bilatéral, de préférence la maladie auditive est une perte d'audition unilatérale ou bilatérale avec un seuil audiométrique tonal de 30 dB à 70 dB à 3 fréquences contiguës, optionnellement associée à un acouphène unilatéral ou bilatéral, le seuil audiométrique tonal étant mesuré par la réponse auditive du tronc cérébral ou par l'audiométrie tonale pure.

11. Composé pour utilisation selon l'une quelconque des revendications **1** à **10,** dans lequel le sujet porte au moins un dispositif auditif.

12. Composé pour utilisation selon l'une quelconque des revendications **1** à **11,** dans lequel le composé de formule (I) ou un sel et/ou un solvate pharmaceutiquement acceptable de celui-ci est administré au sujet au cours d'une chirurgie cochléaire, par exemple au cours d'une chirurgie d'implantation cochléaire.

13. Composé pour utilisation selon l'une quelconque des revendications **1** à **12,** dans lequel le sujet est un animal mammifère doté d'un tympan, de préférence ledit animal mammifère doté d'un tympan est choisi dans le groupe constitué par un humain, un chat, un chien et un animal primate non humain, plus préférentiellement ledit animal mammifère doté d'un tympan est un chien.

14. Composition pharmaceutique transtympanique comprenant un composé de formule (I) ou un sel et/ou un solvate pharmaceutiquement acceptable de celui-ci tels que définis dans l'une quelconque des revendications **1** à **5** et une combinaison d'un mélange de triglycérides polyoxyéthylés et de poloxamères, ladite composition pharmaceutique étant un gel thermoréversible.

15. Composition pharmaceutique pour utilisation dans le traitement d'une maladie auditive chez un sujet, ladite composition pharmaceutique comprenant un composé de formule (I) ou un sel et/ou un solvate pharmaceutiquement acceptable de celui-ci selon l'une quelconque des revendications **1** à **5** et au moins un excipient pharmaceutiquement acceptable, de préférence ladite maladie auditive étant telle que définie dans l'une quelconque des revendications **8** à **10.**
